# EUROPEAN PATENT SPECIFICATION

(11) **EP 3 648 828 B1**
(45) Date of publication and mention of the grant of the patent: **14.09.2022**
(21) Application number: 18829034.0
(22) Date of filing: 29.06.2018
(51) Int. Cl.: A61M 25/00, A61M 25/01, A61M 25/06, A61M 25/09, A61M 25/098

(54) **METHODS AND DEVICES FOR TRANSCAROTID ACCESS**
VERFAHREN UND VORRICHTUNGEN FÜR TRANSKAROTISZUGANG
MÉTHODES ET DISPOSITIFS D'ACCÈS TRANS-CAROTIDIEN

(30) Priority: 05.07.2017 US 201715641966
(43) Date of publication of application: 13.05.2020
(73) Proprietor: Silk Road Medical, Inc., Sunnyvale, California 94089 (US)
(72) Inventor: KUME, Stewart, M., Sunnyvale, CA 94089 (US); CHANG, Michael, Sunnyvale, CA 94089 (US); WALLACE, Michael, P., Sunnyvale, CA 94089 (US)
(74) Representative: Slingsby Partners LLP
(86) International application number: PCT/US2018/040264
(87) International publication number: WO 2019/010077

(56) References cited:
- US-A- 3 993 079
- US-A- 3 993 079
- US-A- 5 084 022
- US-A1- 2004 181 150
- US-A1- 2005 107 820
- US-A1- 2008 255 475
- US-A1- 2008 255 475
- US-A1- 2008 306 408
- US-A1- 2016 158 502
- US-A1- 2016 158 502

## Description

### BACKGROUND

The present disclosure relates generally to medical systems, and devices for performing endovascular interventions. More particularly, the present disclosure relates to systems for access directly into the carotid artery to perform interventional procedures in the treatment of vascular disease and other diseases associated with the vasculature.

Interventional procedures are performed to treat vascular disease, for example stenosis, occlusions, aneurysms, or fistulae. Interventional procedures are also used to perform procedures on organs or tissue targets that are accessible via blood vessels, for example denervation or ablation of tissue to intervene in nerve conduction, embolization of vessels to restrict blood flow to tumors or other tissue, and delivery of drugs, contrast, or other agents to intra or extravascular targets for therapeutic or diagnostic purposes. Interventional procedures are typically divided into coronary, neurovascular, and peripheral vascular categories. Most procedures are performed in the arterial system via an arterial access site.

Methods for gaining arterial access to perform these procedures are well-established, and fall into two broad categories: percutaneous access and surgical cut-down. The majority of interventional procedures utilize a percutaneous access. For this access method, a needle puncture is made from the skin, through the subcutaneous tissue and muscle layers to the vessel wall, and into the vessel itself. Vascular ultrasound is often used to image the vessel and surrounding structures, and facilitate accurate insertion of the needle into the vessel. Depending on the size of the artery and of the access device, the method will vary, for example a Seldinger technique or modified Seldinger technique consists of placing a sheath guide wire through the needle into the vessel. Typically the sheath guide wire is 0.889 mm or 0.965 mm (0.035" or 0.038"). In some instances, a micro-puncture or micro access technique is used whereby the vessel is initially accessed by a small gauge needle, and successively dilated up by a 4F micropuncture cannula through which the sheath guidewire is placed. Once the guidewire is placed, an access sheath and sheath dilator are inserted over the guide wire into the artery. In other instances, for example if a radial artery is being used as an access site, a smaller sheath guidewire is used through the initial needle puncture, for example a 0.457 mm (0.018") guidewire. The dilator of a radial access sheath is designed to accommodate this smaller size guidewire, so that the access sheath and dilator can be inserted over the 0.457 mm (0.018") wire into the artery.

In a surgical cut-down, a skin incision is made and tissue is dissected away to the level of the target artery. This method is often used if the procedure requires a large access device, if there is risk to the vessel with a percutaneous access, and/or if there is possibility of unreliable closure at the access site at the conclusion of the procedure. Depending on the size of the artery and of the access device, an incision is made into the wall of the vessel with a blade, or the vessel wall is punctured directly by an access needle, through which a sheath guide wire is placed. The micropuncture technique may also be used to place a sheath guide wire. As above, the access sheath and sheath dilator are inserted into the artery over the sheath guide wire. Once the access sheath is placed, the dilator and sheath guide wire are removed. Devices can now be introduced via the access sheath into the artery and advanced using standard interventional techniques and fluoroscopy to the target site to perform the procedure.

Access to the target site is accomplished from an arterial access site that is easily entered from the skin. Usually this is the femoral artery which is both relatively large and relatively superficial, and easy to close on completion of the procedure using either direct compression or one of a variety of vessel closure devices. For this reason, endovascular devices are specifically designed for this femoral access site. However, the femoral artery and its vicinity are sometimes diseased, making it difficult or impossible to safely access or introduce a device into the vasculature from this site. In addition, the treatment target site may be quite some distance from the femoral access point requiring devices to be quite lengthy and cumbersome. Further, reaching the target site from the femoral access point may involve traversing tortuous and/or diseased arteries, which adds time and risk to the procedure. For these reasons, alternate access sites are sometimes employed. These include the radial, brachial and axillary arteries. However, these access sites are not always ideal, as they involve smaller arteries and may also include tortuous segments and some distance between the access and target sites.

### Some Exemplary Issues with Current Technology

In some instances, a desired access site is the carotid artery. For example, procedures to treat disease at the carotid artery bifurcation and internal carotid artery are quite close to this access site. Procedures in the intracranial and cerebral arteries are likewise much closer to this access site than the femoral artery. This artery is also larger than some of the alternate access arteries noted above. (The common carotid artery is typically 6 to 10 mm in diameter, the radial artery is 2 to 3 mm in diameter.)

Because most access devices used in interventional procedure are designed for the femoral access, these devices are not ideal for the alternate carotid access sites, both in length and mechanical properties. This makes the procedure more cumbersome and in some cases more risky if using devices designed for femoral access in a carotid access procedure. For example, in some procedures it is desirable to keep the distal tip of the access sheath below or away from the carotid bifurcation, for example in procedures involving placing a stent at the carotid bifurcation. For patients with a low bifurcation, a short neck, or a very deep carotid artery, the angle of entry of the sheath into the artery (relative to the longitudinal axis of the artery) is very acute with respect to the longitudinal axis of the artery, i.e. more perpendicular than parallel relative to the longitudinal axis of the artery. This acute angle increases the difficulty and risk in sheath insertion and in insertion of devices through the sheath. In these procedures, there is also risk of the sheath dislodgement as only a minimal length of sheath can be inserted. In femoral or radial access cases, the sheaths are typically inserted into the artery all the way to the hub of the sheath, making sheath position very secure and parallel to the artery, so that the issues with steep insertion angle and sheath dislodgement do not occur in femoral or radial access sites.

In other procedures, it is desirable to position the sheath tip up to and possibly including the petrous portion of the internal carotid artery, for example in procedures requiring access to cerebral vessels. Conventional interventional sheaths and sheath dilators are not flexible enough to be safely positioned at this site.

In addition, radiation exposure may be a problem for the hands of the operators for procedures utilizing a transcarotid access site, if the working areas are close to the access site.

Document US2016/158502 A1 discloses a micropuncture access needle comprising a proximal hub coupled to an elongate shaft defining an inner lumen, an access guidewire sized to be received through the inner lumen of the micropuncture access needle, the guidewire comprising a distal tip and at least one depth indicator positioned on the access guidewire a distance away from the distal tip of the guidewire; and a microaccess cannula comprising an elongate body defining an inner lumen and a depth indicator formed on the elongate body.

### SUMMARY

What is needed is a system of devices that optimize ease and safety of arterial access directly into the common carotid artery. What is also needed is a system of devices which minimize radiation exposure to the operator. What are also needed are methods for safe and easy access into the carotid artery to perform peripheral and neurovascular interventional procedures.

Disclosed are methods and devices that enable safe, rapid and relatively short and straight transcarotid access to the arterial vasculature to treat coronary, peripheral and neurovascular disease states. The devices and associated methods include transcarotid access devices, guide catheters, catheters, and guide wires specifically to reach a target anatomy via a transcarotid access site. Included in this disclosure are kits of various combinations of these devices to facilitate multiple types of transcarotid interventional procedures.

In one aspect, there is disclosed a system of devices for accessing a carotid artery via a direct puncture of the carotid arterial wall, comprising a sheath guide wire, an arterial access sheath and a sheath dilator, wherein the arterial access sheath and sheath dilator are sized and configured to be inserted in combination over the sheath guide wire directly into the common carotid artery, and wherein the sheath has an internal lumen and a proximal port such that the lumen provides a passageway for an interventional device to be inserted via the proximal port into the carotid artery.

In another aspect, the system for accessing a carotid artery also includes: an access needle, an access guide wire, and an access cannula, all sized and configured to insert a sheath guide wire through the wall of the carotid artery so that the arterial access sheath and dilator may be placed into the carotid artery either percutaneously or via a surgical cut down.

In another aspect, there is disclosed a method for treatment of coronary, peripheral or neurovascular disease, comprising: forming a penetration in a wall of a carotid artery; positioning an arterial access sheath through the penetration into the artery; and treating a target site using a treatment device.

In another aspect, there is disclosed an arterial access sheath for introducing an interventional device into an artery. The arterial access sheath includes an elongated body sized and shaped to be transcervically introduced into a common carotid artery at an access location in the neck and an internal lumen in the elongated body having a proximal opening in a proximal region of the elongated body and a distal opening in a distal region of the elongated body. The internal lumen provides a passageway for introducing an interventional device into the common carotid artery when the elongated body is positioned in the common carotid artery. The elongated body has a proximal section and a distalmost section that is more flexible than the proximal section. A ratio of an entire length of the distalmost section to an overall length of the sheath body is one tenth to one half the overall length of the sheath body.

In an interrelated aspect, there is disclosed a micropuncture kit for direct access into a lumen of a surgically exposed vessel using direct visual guidance. The kit includes a micropuncture access needle having a proximal hub coupled to an elongate shaft defining an inner lumen and a visible depth indicator positioned on the elongate shaft a distance away from a distal tip of the elongate shaft. The kit includes an access guidewire sized to be received through the inner lumen of the micropuncture access needle. The guidewire includes a distal tip and at least one visible depth indicator positioned on the access guidewire a distance away from the distal tip of the guidewire. The kit includes a microaccess cannula having an elongate body defining an inner lumen and a plurality of visible depth indicators formed on the elongate body, wherein each of the plurality of visible depth indicators identifies a distance from a distal tip of the cannula.

The access guidewire can include a distalmost flexible section including the distal tip of the guidewire, a transition section proximal to the distalmost flexible section, and a stiffer core section extending proximally from the transition section. The distalmost flexible section can be between 1 cm and 2 cm, and the transition section can be between 2 cm and 3 cm. The transition section and the core section can be configured to support the microaccess cannula inserted into the vessel. The guidewire can be in a range of 0.356 mm to 0.457 mm (0.014" to 0.018") outer diameter and the micropuncture needle can be in a range from 0.80 mm to 0.55 mm (21G to 24G). The at least one visible depth indicator positioned on the access guidewire can have a proximal edge, a distal edge and a width extending between the proximal edge and the distal edge. The distance away from the distal tip of the guidewire can be measured from the distal tip to the distal edge of the at least one visible depth indicator. Inserting the access guidewire through the inner lumen of the elongate shaft until the distal edge is aligned with a back end of the proximal hub of the access needle can extend the distal tip of the guidewire beyond the distal tip of the elongate shaft an extension length. Advancing the access guidewire through the inner lumen of the elongate shaft until the proximal edge is aligned with the back end of the proximal hub of the access needle can extend the distal tip of the guidewire beyond the distal tip of the elongate shaft the extension length plus the width of the at least one visible depth indicator. The extension length can be 3 cm and the width of the at least one visible depth indicator can be 2 cm. The visible depth indicator of the access needle can be a chemical-etched marker, a laser-etched marker, or a pad printed marker. Each of the plurality of visible depth indicators can be formed by a number of marks. The number of marks can identify a number of increments from the distal tip of the cannula. Each increment can be 10 mm and the number of marks can be at least one mark. A first of the plurality of visible depth indicators on the cannula can include one mark and the first visible depth indicator can be 10 mm away from the distal tip of the cannula. A second of the plurality of visible depth indicators on the cannula can be two marks and the second visible depth indicator can be 20 mm away from the distal tip of the cannula. A third of the plurality of visible depth indicators on the cannula can be three marks and the third visible depth indicator can be 30 mm away from the distal tip of the cannula. A fourth of the plurality of visible depth indicators on the cannula can be four marks and the fourth visible depth indicator can be 40 mm away from the distal tip of the cannula. A fifth of the plurality of visible depth indicators on the cannula can be 50 mm away from the distal tip of the cannula and can be a solid band having a width that is greater than a width of one mark. The distance the visible depth indicator of the access needle is from the distal tip of the elongate shaft can be between 3 mm and 7 mm. The visible depth indicator of the access needle can be a chemical-etched marker, a laser-etched marker, or a pad printed marker.

Other features and advantages should be apparent from the following description of various embodiments, which illustrate, by way of example, the principles of the invention.

### BRIEF DESCRIPTION OF THE DRAWINGS

FIG. 1 shows a transcarotid initial access system.
FIG. 2 shows a transcarotid access sheath system.
FIG. 3 shows a component of the transcarotid access system being used to access a carotid artery for a carotid artery procedure.
FIG. 4 shows an access sheath of the transcarotid access system being used to access an internal carotid artery for an intracranial or neurovascular procedure.
FIG. 5 shows an embodiment of an arterial access sheath.
FIGs. 6 and 7 show distal regions of an arterial access sheath.
FIGs. 8-1 IE show embodiments of an arterial access sheath.
FIG. 12 shows an embodiment of a dilator.
FIGs. 13 and 14 show enlarged views of the proximal region of the dilator.
FIGs. 15 and 16 show embodiments of a two part dilator.
FIG. 17 shows a distal region of a dilator having two guidewire lumens.
FIG. 18 shows a cross-sectional view of the distal region of FIG. 17.
FIG. 19A shows an implementation of a transcarotid initial access system.
FIG. 19B shows a detail view of the access needle of FIG. 19A taken at circle B-B.
FIG. 20A illustrates a distal end region of an implementation of an access guidewire.
FIGs. 20B-20C illustrate relative extension of the access guidewire of FIG. 20A and the access needle of FIG. 19A.
FIG. 21A is an implementation of a microaccess cannula.
FIG. 21B is a detail view of the microaccess cannula taken at circle B-B of FIG. 21A.

### DETAILED DESCRIPTION

Disclosed are methods, systems, and devices for accessing and treating the vasculature via a transcarotid access point in the region of the carotid artery.

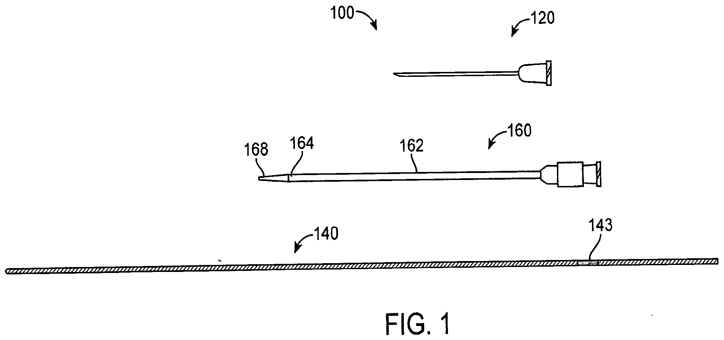

FIG. 1 shows a first embodiment of a transcarotid initial access system 100 of devices for establishing initial access to a carotid artery for the purpose of enabling introduction of a guide wire into the carotid artery. The access to the carotid artery occurs at an access site located in the neck of a patient such as in the region of the patient's carotid artery. The devices of the transcarotid initial access system 100 are particularly suited for directly accessing the carotid artery through the wall of the common carotid artery.

As shown in FIG. 1, the transcarotid initial access system 100 includes an access needle 120, access guidewire 140, and micropuncture cannula 160. The access needle 120, access guidewire 140, and micropuncture cannula 160 are all adapted to be introduced via a carotid puncture into the carotid artery as further described below. The carotid puncture may be accomplished, for example, percutaneously or via a surgical cut down. Embodiments of the initial access system 100 may be adapted towards one or the other method of puncture, as further described below.

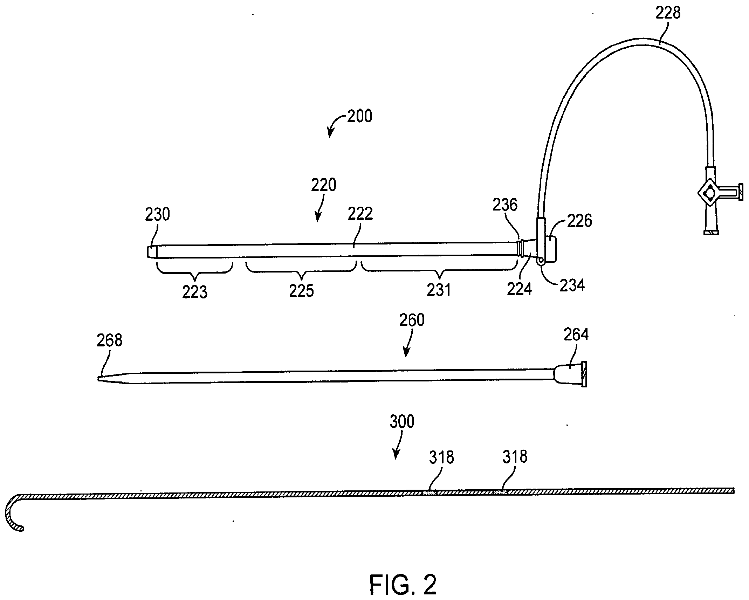

Upon establishment of access to the carotid artery using the initial access system 100, an access sheath may be inserted into the carotid artery at the access site wherein the access sheath may be part of a transcarotid access sheath system. FIG. 2 shows a first embodiment of a transcarotid access sheath system 200 of devices for inserting an access sheath into the carotid artery over a sheath guidewire. When inserted into the carotid artery, the access sheath enables or allows introduction of at least one interventional device into the carotid artery via a lumen of the access sheath for the purpose of performing an interventional procedure on a region of the vasculature. The transcarotid access sheath system 200 includes an access sheath 220, a sheath dilator 260, and a sheath guidewire 300. The access sheath 220, sheath dilator 260 and sheath guidewire 300 are all adapted to be introduced via a carotid puncture into the carotid artery as further described below. The carotid puncture may be accomplished percutaneously or via a surgical cut down. Embodiments of the system 200 may be adapted towards one or the other method of puncture, as further described below.

In an embodiment, some or all of the components of transcarotid initial access system 100 and the transcarotid access sheath system 200 may be combined into one transcarotid access system kit such as by combining the components into a single, package, container or a collection of containers that are bundled together.

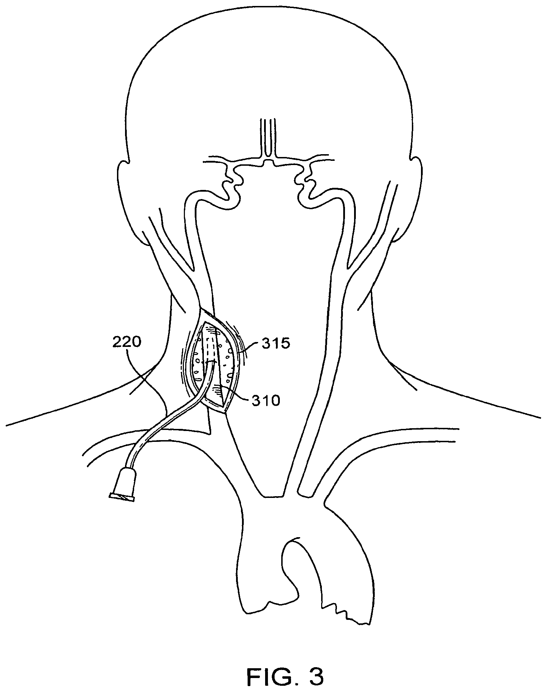

FIG. 3 shows the access sheath 220 being used to access a common carotid artery 310 for a carotid stenting procedure. The access sheath 220 is inserted into the common carotid artery 310 via a surgical cut down 315. As described further below, the access sheath 220 has an internal lumen with openings at proximal and distal tips or regions of the access sheath 220. With a distal portion of the access sheath 220 in the carotid artery and a proximal portion external to the patient, the internal lumen provides a passageway to insert an interventional device into the artery.

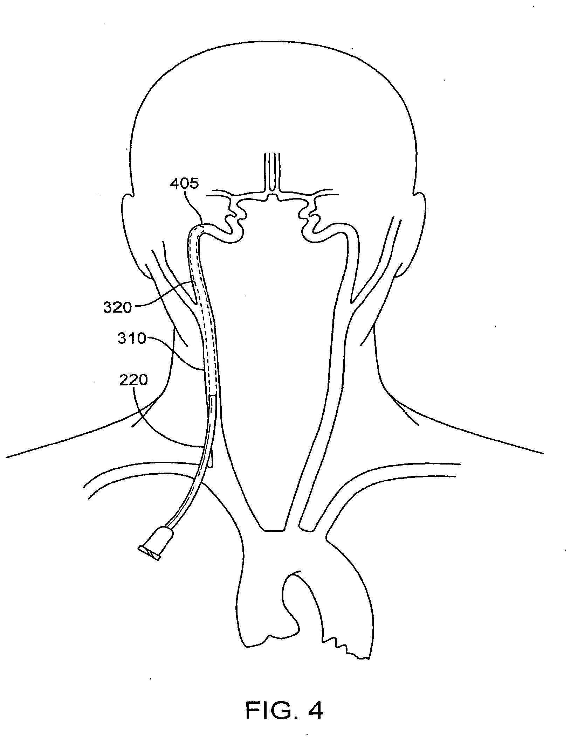

FIG. 4 shows an access sheath 200 of the transcarotid access system being used to access an internal carotid artery 405 for an intracranial or neurovascular procedure. The arterial access sheath 200 accesses the common carotid artery 310 via insertion through a transcervical puncture. Once inserted into the common carotid artery 310, the distal tip of the access sheath 220 is advanced into the internal carotid artery ICA 320 and upward (relative to the puncture in FIG. 4) toward distal cervical or petrous ICA 405 or beyond.

FIGs. 3 and 4 both show the arterial access sheath 220 being advanced upward through the patient's neck toward the patient's brain. In another embodiment, the arterial access sheath 220 may be advanced downward (relative to access locations in FIGs. 3-4) toward the patient's heart such as toward the aorta for example. U.S. Patent No. 8,545,552 entitled "Systems and Methods for Transcatheter Aortic Valve Treatment" describes exemplary methods of directly inserting an access sheath into the carotid artery and advancing an interventional device toward the aorta and ultimately towards the aortic valve.

### Arterial access sheath

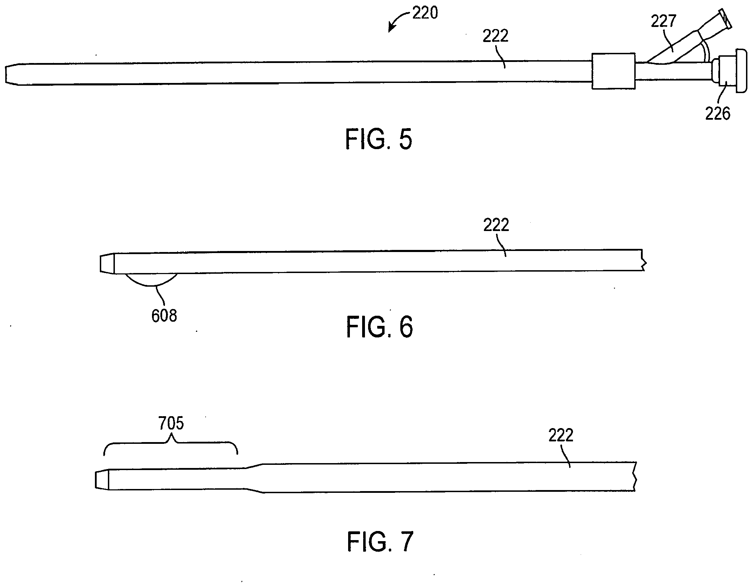

With reference again to FIG. 2, an embodiment of a transcarotid arterial access sheath 220 includes an elongated sheath body 222 and a proximal adaptor 224 at a proximal end of the elongated sheath body 222 of the access sheath 220. The elongated sheath body 222 is the portion of the arterial access sheath 220 that is sized and shaped to be inserted into the artery and wherein at least a portion of the elongated sheath body is actually inserted into the artery during a procedure. The proximal adaptor 224 includes a hemostasis valve 226 and an elongated flush line 228 having an internal lumen that communicates with an internal lumen of the sheath body 222. The proximal adaptor 224 may have a larger diameter or cross-sectional dimension than the sheath body 222. The hemostasis valve 226 communicates with the internal lumen of the sheath body 222 to allow introduction of devices therein while preventing or minimizing blood loss via the internal lumen during the procedure. In an embodiment, the hemostasis valve 226 is a static seal-type passive valve. In an alternate embodiment of the arterial access sheath 220 (shown in FIG. 5) the hemostasis valve 226 is an adjustable-opening valve such as a Tuohy-Borst valve 227 or rotating hemostasis valve (RHV). Alternately, the access sheath 220 may terminate on the proximal end in a female Luer adaptor to which a separate hemostasis valve component may be attached, either a passive seal valve, a Tuohy-Borst valve or rotating hemostasis valve (RHV).

The elongated sheath body 222 of the arterial access sheath 220 has a diameter that is suitable or particularly optimized to provide arterial access to the carotid artery. In an embodiment, the elongated sheath body 222 is in a size range from 5 to 9 French, or alternately in an inner diameter range from 0.072 inches to .126 inches. In an embodiment, the elongated sheath body 222 is a 6 or 7 French sheath. In an embodiment where the sheath is also used for aspiration or reverse flow, or to introduce larger devices, the sheath is an 8 French sheath.

The elongated sheath body 222 of the arterial access sheath 220 has a length from the proximal adapter 224 to a distal tip of the elongated sheath body 222 that is suitable for reaching treatment sites located in or toward the brain relative to an arterial access site in the common carotid artery CCA. For example, to access a carotid artery bifurcation or proximal internal carotid artery ICA from a CCA access site, the elongated sheath body 222 (i.e., the portion that can be inserted into the artery) of the access sheath 220 may have a length in a range from 7 to 15 cm. In an embodiment, the elongated sheath body 222 has a length in the range of 10-12 cm. For access to a same target site from a femoral access site, typical access sheaths must be between 80 and 110 cm, or a guide catheter must be inserted through an arterial access sheath and advanced to the target site. A guide catheter through an access sheath takes up luminal area and thus restricts the size of devices that may be introduced to the target site. Thus an access sheath that allows interventional devices to reach a target site without a guide catheter has advantages over an access sheath that requires use of a guide catheter to allow interventional devices to the target site.

Alternately, to position the distal tip of the elongated sheath body 222 more distally relative to the access site, for example to perform an intracranial or neurovascular procedure from a CCA access site, the elongated sheath body 222 of the access sheath 220 may have a length in the range from 10 cm to 30 cm, depending on the desired target position of the sheath distal tip. For example, if the target position is the distal CCA or proximal ICA, the elongated sheath body 222 may be in the range from 10 cm to 15 cm. If the desired target position is the mid to distal cervical, petrous, or cavernous segments of the ICA, the elongated sheath body 222 may be in the range from 15 to 30 cm.

Alternately, the arterial access sheath 220 is configured or adapted for treatment sites or target locations located proximal to the arterial access site (i.e. towards the aorta) when the access site is in the common carotid artery. For example the treatment site may be the proximal region of the CCA, CCA ostium, ascending or descending aorta or aortic arch, aortic valve, coronary arteries, or other peripheral arteries. For these target locations, the appropriate length of the elongated sheath body 222 depends on the distance from the target location to the access site. In this configuration, the elongated sheath body 222 is placed through an arterial access site and directed inferiorly towards the aorta.

The access sheath 220 may also include a radiopaque tip marker 230. In an example the radiopaque tip marker is a metal band, for example platinum iridium alloy, embedded near the distal end of the sheath body 222 of the access sheath 220. Alternately, the access sheath tip material may be a separate radiopaque material, for example a barium polymer or tungsten polymer blend. The sheath tip itself is configured such that when the access sheath 220 is assembled with the sheath dilator 260 to form a sheath assembly, the sheath assembly can be inserted smoothly over the sheath guide wire 300 through the arterial puncture with minimal resistance. In an embodiment, the elongated sheath body 222 of the access sheath 220 has a lubricious or hydrophilic coating to reduce friction during insertion into the artery. In an embodiment, the distal coating is limited to the distalmost 0.5 to 3 cm of the elongated sheath body 222, so that it facilitates insertion without compromising security of the sheath in the puncture site or the ability of the operator to firmly grasp the sheath during insertion. In an alternate embodiment, the sheath has no coating.

With reference to FIG. 2, in an embodiment, the arterial access sheath 220 has features to aid in securement of the sheath during the procedure. For example the access sheath 220 may have a suture eyelet 234 or one or more ribs 236 molded into or otherwise attached to the adaptor 224 (located at the proximal end of the elongated sheath body 222) which would allow the operator to suture tie the sheath hub to the patient.

For a sheath adapted to be inserted into the common carotid artery for the purpose of access to the carotid bifurcation, the length of the elongated sheath body 222 can be in the range from 7 to 15 cm, usually being from 10 cm to 12 cm. The inner diameter is typically in the range from 5 Fr (1 Fr = 0.33 mm), to 10 Fr, usually being 6 to 8 Fr. For a sheath adapted to be inserted via the common carotid artery to the mid or distal internal carotid artery for the purpose of access to the intracranial or cerebral vessels, the length of the elongated sheath body 222 can be in the range from 10 to 30 cm, usually being from 15 cm to 25 cm. The inner diameter is typically in the range from 5 Fr (1 Fr = 0.33 mm), to 10 Fr, usually being 5 to 6 Fr.

Particularly when the sheath is being introduced through the transcarotid approach, above the clavicle but below the carotid bifurcation, it is desirable that the elongated sheath body 222 be flexible while retaining hoop strength to resist kinking or buckling. This is especially important in procedures that have limited amount of sheath insertion into the artery, and there is a steep angle of insertion as with a transcarotid access in a patient with a deep carotid artery and/or with a short neck. In these instances, there is a tendency for the sheath body tip to be directed towards the back wall of the artery due to the stiffness of the sheath. This causes a risk of injury from insertion of the sheath body itself, or from devices being inserted through the sheath into the arteries, such as guide wires. Alternately, the distal region of the sheath body may be placed in a distal carotid artery which includes one or more bends, such as the petrous ICA. Thus, it is desirable to construct the sheath body 222 such that it can be flexed when inserted in the artery, while not kinking. In an embodiment, the sheath body 222 is circumferentially reinforced, such as by stainless steel or nitinol braid, helical ribbon, helical wire, cut stainless steel or nitinol hypotube, cut rigid polymer, or the like, and an inner liner so that the reinforcement structure is sandwiched between an outer jacket layer and the inner liner. The inner liner may be a low friction material such as PTFE. The outer jacket may be one or more of a group of materials including Pebax, thermoplastic polyurethane, or nylon.

In an embodiment, the sheath body 222 may vary in flexibility over its length. This change in flexibility may be achieved by various methods. For example, the outer jacket may change in durometer and/or material at various sections. Alternately, the reinforcement structure or the materials may change over the length of the sheath body. In one embodiment, there is a distalmost section of sheath body 222 which is more flexible than the remainder of the sheath body. For example, the flexural stiffness of the distalmost section is one third to one tenth the flexural stiffness of the remainder of the sheath body 222. In an embodiment, the distalmost section has a flexural stiffness (E^{∗}I) in the range 50 to 300 N-mm² and the remaining portion of the sheath body 222 has a flexural stiffness in the range 500 to 1500 N-mm², where E is the elastic modulus and I is the area moment of inertia of the device. For a sheath configured for a CCA access site, the flexible, distal most section comprises a significant portion of the sheath body 222 which may be expressed as a ratio. In an embodiment, the ratio of length of the flexible, distalmost section to the overall length of the sheath body 222 is at least one tenth and at most one half the length of the entire sheath body 222.

In some instances, the arterial access sheath is configured to access a carotid artery bifurcation or proximal internal carotid artery ICA from a CCA access site. In this instance, an embodiment of the sheath body 222 has a distalmost section 223 which is 3 to 4 cm and the overall sheath body 222 is 10 to 12 cm. In this embodiment, the ratio of length of the flexible, distalmost section to the overall length of the sheath body 222 is about one forth to one half the overall length of the sheath body 222. In another embodiment, there is a transition section 225 between the distalmost flexible section and the proximal section 231, with one or more sections of varying flexibilities between the distalmost section and the remainder of the sheath body. In this embodiment, the distalmost section is 2 to 4 cm, the transition section is 1 to 2 cm and the overall sheath body 222 is 10 to 12 cm, or expressed as a ratio, the distalmost flexible section and the transition section collectively form at least one fourth and at most one half the entire length of the sheath body.

In some instances, the sheath body 222 of the arterial access sheath is configured to be inserted more distally into the internal carotid artery relative to the arterial access location, and possibly into the intracranial section of the internal carotid artery. For example, a distalmost section 223 of the elongated sheath body 222 is 2.5 to 5 cm and the overall sheath body 222 is 20 to 30 cm in length. In this embodiment, the ratio of length of the flexible, distalmost section to the overall length of the sheath body is one tenth to one quarter of the entire sheath body 222. In another embodiment, there is a transition section 225 between the distalmost flexible section and the proximal section 231, in which the distalmost section is 2.5 to 5 cm, the transition section is 2 to 10 cm and the overall sheath body 222 is 20 to 30 cm. In this embodiment, the distalmost flexible section and the transition section collectively form at least one sixth and at most one half the entire length of the sheath body.

Other embodiments are adapted to reduce, minimize or eliminate a risk of injury to the artery caused by the distal-most sheath tip facing and contacting the posterior arterial wall. In some embodiments, the sheath has a structure configured to center the sheath body tip in the lumen of the artery such that the longitudinal axis of the distal region of the sheath body is generally parallel with the longitudinal or center axis of the lumen of the vessel. In an embodiment shown in FIG. 6, the sheath alignment feature is an inflatable or enlargeable bumper, for example a balloon 608, located on an outer wall of the arterial access sheath 220. The balloon 608 may be increased in size to exert a force on inner the arterial that contacts and pushes the elongated body 222 of the aerial access sheath away from the arterial wall.

In another embodiment, the sheath alignment feature is one or more mechanical structures on the sheath body that can be actuated to extend outward from the sheath tip. In an embodiment, the sheath body 222 is configured to be inserted into the artery such that a particular edge of the arterial access is against the posterior wall of the artery. In this embodiment, the sheath alignment feature need only extend outward from one direction relative to the longitudinal axis of the sheath body 222 to lift or push the sheath tip away from the posterior arterial wall. For example, as shown in FIG. 6, the inflatable bumper 608 is a blister on one side of the sheath body. In another example, the mechanical feature extends only on one side of the sheath body.

In another embodiment, at least a portion of the sheath body 222 is pre-shaped so that after sheath insertion the tip is more aligned with the long axis of the vessel, even at a steep sheath insertion angle. In this embodiment the sheath body is generally straight when the dilator is assembled with the sheath during sheath insertion over the sheath guide wire, but once the dilator and guidewire are removed, the distalmost section of the sheath body assumes a curved or angled shape. In an embodiment, the sheath body is shaped such that the distalmost 0.5 to 1 cm section is angled from 10 to 30 degrees, as measured from the main axis of the sheath body, with a radius of curvature about 12.7 mm (0.5"). To retain the curved or angled shape of the sheath body after having been straightened during insertion, the sheath may be heat set in the angled or curved shape during manufacture. Alternately, the reinforcement structure may be constructed out of nitinol and heat shaped into the curved or angled shape during manufacture. Alternately, an additional spring element may be added to the sheath body, for example a strip of spring steel or nitinol, with the correct shape, added to the reinforcement layer of the sheath.

In an alternate embodiment, there are procedures in which it is desirable to minimize flow resistance through the access sheath such as described in U.S. Patent No. 7,998,104 to Chang and U.S. Patent No. 8,157,760 to Criado. FIG. 7 shows such an embodiment of the sheath body 222 where the sheath body has stepped or tapered configuration having a reduced diameter distal region 705 (with the reduced diameter being relative to the remainder of the sheath). The distal region 705 of the stepped sheath can be sized for insertion into the carotid artery, typically having an inner diameter in the range from 1.651 mm to 2.921 mm (0.065" to 0.115") with the remaining proximal region of the sheath having larger outside and luminal diameters, with the inner diameter typically being in the range from 2.794 mm to 3.429 mm (0.110" to 0.135"). The larger luminal diameter of the remainder of the sheath body minimizes the overall flow resistance through the sheath. In an embodiment, the reduced-diameter distal section 705 has a length of approximately 2 cm to 4 cm. The relatively short length of the reduced-diameter distal section 705 permits this section to be positioned in the common carotid artery CCA via a transcarotid approach with reduced risk that the distal end of the sheath body will contact the bifurcation B. Moreover, the reduced diameter section also permits a reduction in size of the arteriotomy for introducing the sheath into the artery while having a minimal impact in the level of flow resistance. Further, the reduced distal diameter section may be more flexible and thus more conformal to the lumen of the vessel.

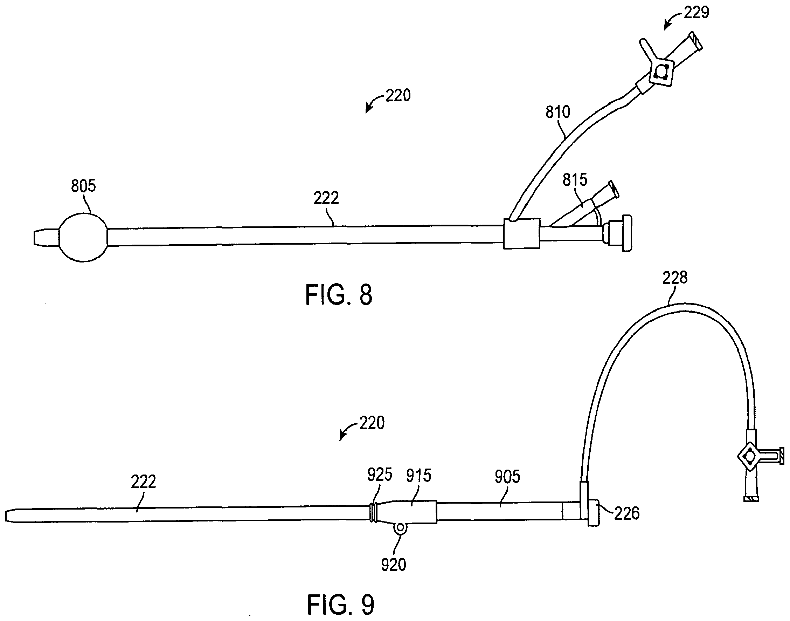

In some instances it is desirable for the sheath body 222 to also be able to occlude the artery in which it is positioned, for examples in procedures that may create distal emboli. In these cases, occluding the artery stops antegrade blood flow in the artery and thereby reduces the risk of distal emboli that may lead to neurologic symptoms such as TIA or stroke. FIG. 8 shows an embodiment of an arterial access sheath 220 with an inflatable balloon 805 on a distal region that is inflated via an inflation line 810 that connects an internal inflation lumen in the sheath body 222 to a stopcock 229 which in turn may be connected to an inflation device. In this embodiment, there is also a Y-arm 815 that may be connected to a passive or active aspiration source to further reduce the risk of distal emboli.

In some instances it is desirable to move the hemostasis valve away from the distal tip of the sheath, while maintaining the length of the insertable sheath body 222 of the sheath. This embodiment is configured to move the hands of the operator, and in fact his or her entire body, away from the target site and therefore from the image intensifier that is used to image the target site fluoroscopically, thus reducing the radiation exposure to the user during the procedure. Essentially, this lengthens the portion of the arterial access sheath 220 that is outside the body. This portion can be a larger inner and outer diameter than the sheath body 222. In instances where the outer diameter of the catheter being inserted into the sheath is close to the inner diameter of the sheath body, the annular space of the lumen that is available for flow is restrictive. Minimizing the sheath body length is thus advantageous to minimize this resistance to flow, such as during flushing of the sheath with saline or contrast solution, or during aspiration or reverse flow out of the sheath. In an embodiment, as shown in FIG. 9, the arterial access sheath 220 has an insertable, elongated sheath body 222 (i.e. the portion configured to insert into the artery) and a proximal extension portion 905. In an embodiment, the sheath body 222 has an inner diameter of about 2.210 mm (0.087") and an outer diameter of about 2.642 mm (.104"), corresponding to a 6 French sheath size, and the proximal extension has an inner diameter of about 2.540 mm to 3.175 mm (.100" to .125") and an outer diameter of about 3.810 mm to 4.445 mm (.150" to .175"). In another embodiment, the sheath body 222 has an inner diameter of about 2.870 mm (.113") and an outer diameter of about 3.454 mm (.136"), corresponding to an 8 French sheath size, and the proximal extension has an inner diameter of about 3.175mm (.125") and an outer diameter of about 4.445 mm (.175"). In yet another embodiment, the sheath body 222 is stepped with a smaller diameter distal section 705 to further reduce flow restriction, as in FIG. 7. In an embodiment, the proximal extension 905 is a length suitable to meaningfully reduce the radiation exposure to the user during a transcarotid access procedure. For example, the proximal extension 905 is between 10 and 25 cm, or between 15 and 20 cm. Alternately, the proximal extension 905 has a length configured to provide a distance of between about 30 cm and 60 cm between the hemostasis valve 226 and the distal tip of the sheath body, depending on the insertable length of the access sheath. A connector structure 915 can connect the elongated sheath body 222 to the proximal extension 905. In this embodiment, the connector structure 915 may include a suture eyelet 920 and/or ribs 925 to assist in securing the access sheath to the patient. In an embodiment, the hemostasis valve 226 is a static seal-type passive valve. In an alternate embodiment the hemostasis valve 226 is an adjustable-opening valve such as a Tuohy-Borst valve 227 or rotating hemostasis valve (RHV). Alternately, the proximal extension may terminate on the proximal end in a female Luer adaptor to which a separate hemostasis valve component may be attached, either a passive seal valve, a Tuohy-Borst valve or rotating hemostasis valve (RHV).

Typically, vessel closure devices require an arterial access sheath with a maximum distance of about 15 cm between distal tip of the sheath body to the proximal aspect of the hemostasis valve, with sheath body 222 of about 11 cm and the remaining 4 cm comprising the length of the proximal hemostasis valve; thus if the access sheath has a distance of greater than 15 cm it is desirable to remove the proximal extension 905 at the end of the procedure. In an embodiment, the proximal extension 905 is removable in such a way that after removal, hemostasis is maintained. For example a hemostasis valve is built into the connector 915 between the sheath body 222 and the proximal extension 905. The hemostasis valve is opened when the proximal extension 905 is attached to allow fluid communication and insertion of devices, but prevents blood flowing out of the sheath when the proximal extension 905 is removed. After the procedure is completed, the proximal extension 905 can be removed, reducing the distance between the proximal aspect of the hemostasis valve and sheath tip from greater than 15 cm to equal or less than 15 cm and thus allowing a vessel closure device to be used with the access sheath 220 to close the access site.

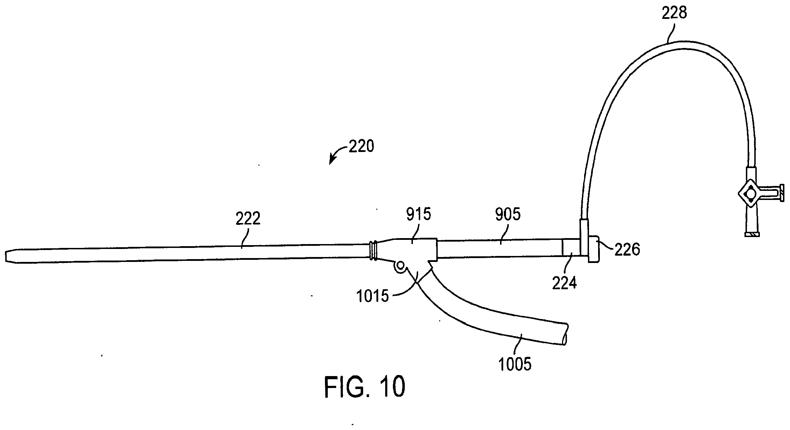

In some procedures it may be desirable to have a low resistance (large bore) flow line or shunt connected to the access sheath, such as described in U.S. Patent No. 7,998,104 to Chang and U.S. Patent No. 8,157,760 to Criado. The arterial sheath embodiment shown in FIG. 10 has a flow line 1005 with internal lumen to a Y-arm 1015 of the connector 915. This flow line has a lumen fluidly connected to a lumen in the sheath body. The flow line 1005 may be connected to a lower pressure return site such as a venous return site or a reservoir. The flow line 1005 may also be connected to an aspiration source such as a pump or a syringe. In an embodiment, an occlusion element may also be included on the distal end of the sheath body 222, for example an occlusion balloon. This may be desirable in percutaneous procedures, where the vessel cannot be occluded by vascular surgical means such as vessel loops or vascular clamps.

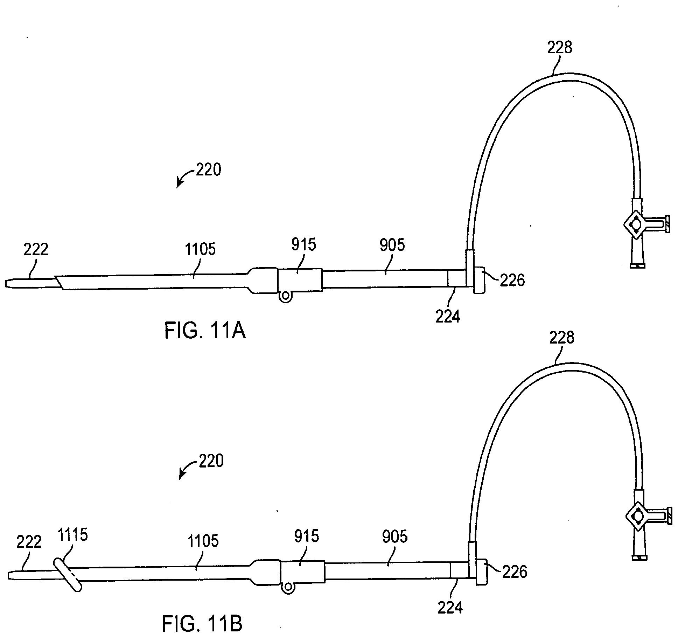

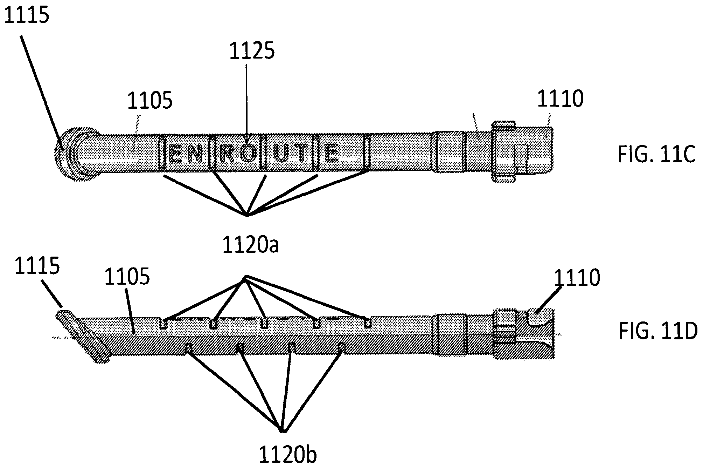

In some procedures, it may be desirable to limit the amount of sheath body insertion into the artery, for example in procedures where the target area is very close to the arterial access site. In a stent procedure of the carotid artery bifurcation, for example, the sheath tip should be positioned proximal of the treatment site (relative to the access location) so that it does not interfere with stent deployment or enter the diseased area and possibly cause emboli to get knocked loose. In an embodiment of arterial sheath 220 shown in FIGs. 11A and 11B, a sheath stopper 1105 is slidably connected or mounted over the outside of the distal portion of the sheath body (see also FIGs. 11C and 11D). The sheath stopper 1105 is shorter than the distal portion of the sheath, effectively shortening the insertable portion of the sheath body 222 by creating a positive stop at a certain length along the sheath body 222. The sheath stopper 1105 may be a tube that slidably fits over the sheath body 222 with a length that, when positioned on the sheath body 222, leaves a distal portion of the sheath body exposed. This length can be in the range 2 to 4 cm. More particularly, the length is 2.5 cm. The distal end of the sheath stopper 1105 may be angled and oriented such that the angle sits flush with the vessel and serves as a stop against the arterial wall when the sheath is inserted into the artery when the vessel is inserted into the artery, as shown in FIG. 11A. Alternately, the distal end of the sheath stopper may be formed into an angled flange 1115 that contacts the arterial wall, as shown in FIG. 11B, 11C, and 11D. The flange 1115 is rounded or has an atraumatic shape to create a more positive and atraumatic stop against the arterial wall. The sheath stopper 1105 may be permanently secured to the arterial sheath, for example the proximal end of the sheath stopper may be adhered to connector 915 of the arterial access sheath. Alternately, the sheath stopper 1105 may be removable from the arterial access sheath 220 by the user so it can be optionally utilized in a procedure. In this instance, the sheath stopper 1105 may have a locking feature 1110 on the proximal portion that engages with a corresponding locking features on the connector 915, for example slots or recesses on the proximal sheath stopper engaging protrusions on the connector. Other locking features may also be utilized.

Again with respect to FIG. 11C-11D, the sheath stopper 1105 can include one or more cutouts, indents, or recessed grooves 1120a or other features along a length of the sheath stopper 1105, for example along the anterior surface of the sheath stopper 1105. The grooves 1120a can be sized to receive sutures used to secure the sheath 220 to the patient to improve sheath stability and mitigate against sheath dislodgement. The one or more recessed grooves 1120a on the sheath stopper 1105 can extend along an arc around the longitudinal axis of the sheath stopper 1105. The one or more recessed grooves 1120a on the anterior surface can alternate or be in a staggered pattern with corresponding one or more recessed grooves 1120b on the posterior surface of the sheath stopper 1105 to provide additional flexibility to the sheath assembly in the anterior-to-posterior plane while maintaining axial strength to allow forward force of the sheath stopper against the arterial wall. Increased flexibility of the sheath assembly can reduce the amount of stress that may be placed on a vessel. In some implementations, a visual indicator 1125 can be positioned on the anterior surface of the sheath stopper 1105. The visual indicator 1125 can be pad-printed text, line, or other pattern. The visual indicator 1125 can be molded directly into the body of the sheath stopper 1105. The indicator 1125 can provide an external visual reference for rotational orientation of the sheath stopper (and thereby orientation of the distal flange 1115) with respect to the vessel. In use, the indicator 1125 can face anteriorly with respect to the patient. Using this orientation, the distal flange 1115 can lie approximately flat against the anterior wall of the vessel, e.g. the common carotid artery. Additional features the sheath stopper 1105 may incorporate are described in U.S. Publication No. 2016/0296690.

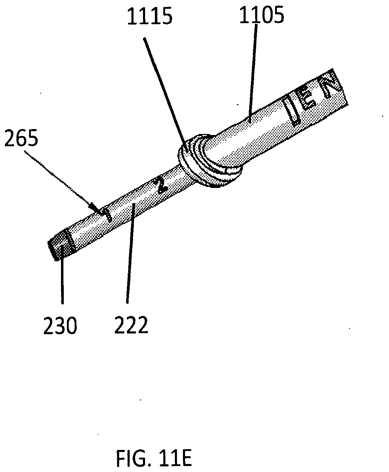

As mentioned above, the access sheath 220 may include a radiopaque tip marker 230. The access sheath 220 can also incorporate one or more markers 265 positioned on the sheath body 222 (see FIG. 11E) providing external visual guidance during insertion of the sheath 220 without special imaging to assess depth of insertion, as will be described in more detail below. In some implementations, the markers 265 can be pad printed markers that designate insertion depths in increments of 1 cm up to about 5 cm. The external visual reference markers 265 on the sheath body 222 can supplement the radiopaque tip marker 230 and the physical depth limit provided by the sheath stopper 1105. The external visual reference markers 265 can be useful, for example, when a user wants to insert the sheath 220 to a shallower depth than prescribed by the sheath stopper 1105 or when a user removes the sheath stopper 1105 from the sheath body 222.

In situations where the insertion of the sheath body is limited to between 2 and 3 cm, and particularly when the sheath body is inserted at a steep angle, the sheath may conform to a bayonet shape when secured to the patient. For example, the bayonet shape may comprise a first portion that extends along a first axis and a second portion that extends along a second axis that is axially offset from the first axis and/or non-parallel to the first axis. The springiness of the sheath body causes this shape to exert a force on the vessel at the site of insertion and increase the tendency of the sheath to come out of the vessel if not properly secured. To reduce the stress on the vessel, the sheath stopper may be pre-shaped into a curved or bayonet shape so that the stress of the sheath body when curved is imparted onto the sheath stopper rather than on the vessel. The sheath stopper may be made from springy but bendable material or include a spring element such as a stainless steel or nitinol wire or strip, so that when the dilator is inserted into the sheath and sheath stopper assembly, the sheath is relatively straight, but when the dilator is removed the sheath stopper assumes the pre-curved shape to reduce the force the sheath imparts on the vessel wall. Alternately, the sheath stopper may be made of malleable material or include a malleable element such as a bendable metal wire or strip, so that it can be shaped after the sheath is inserted into a desired curvature, again to reduce the stress the sheath imparts on the vessel wall.

### Sheath Dilator

With reference again to FIG. 2, the sheath dilator 260 is a component of the transcarotid access sheath system 200. The sheath dilator 260 is an elongated body that is inserted into the artery and enables smooth insertion of the access sheath 220 over the sheath guidewire 300 through a puncture site in the arterial wall. Thus, the distal end of the dilator 260 is generally tapered to allow the dilator to be inserted over the sheath guidewire 300 into the artery, and to dilate the needle puncture site to a larger diameter for insertion of the access sheath 220 itself. To accommodate these functions, the dilator 260 has a tapered end 268 with a taper that is generally between 6 and 12 degrees total included angle (relative to a longitudinal axis of the dilator), with a radiused leading edge. Sheath dilators are typically locked to the access sheath when assembled for insertion into the artery. For example a proximal hub 264 of the sheath dilator 260 is structured to snap into or over a corresponding structure on the hemostasis valve 226 of the arterial access sheath 220. An inner lumen of the dilator 260 accommodates a sheath guidewire 300, with an inner diameter of between 0.940 mm to 1.041 mm (.037 to .041"), depending on the sheath guide wire size for example.

For a transcarotid access sheath system 200, it may be desirable to make the distal section of the sheath dilator 260 more flexible, to correspond with an increased flexible section of the access sheath 220. For example, the distal 2 to 5 cm of the sheath dilator 260 may be 20% to 50% more flexible than the proximal portion of the sheath dilator 260. This embodiment would allow a sheath and dilator being inserted to accommodate a steep insertion angle, as is often the case in a transcarotid access procedure, with a smoother insertion over the guidewire while still maintaining columnar support of the dilator. The columnar support is desirable to provide the insertion force required to dilate the puncture site and insert the access sheath.

For some transcarotid access sheath systems, it may be desirable to also use a smaller diameter access guidewire (for example in the range 0.356 mm to 0.457 mm (.014" to .018" diameter)) to guide the sheath and dilator into the artery. In this embodiment, the sheath dilator tapered end 268 is configured to provide a smooth transition from a smaller wire size to the access sheath. In one variation, the sheath guide wire is 0.457 mm (.018") and the inner dilator lumen is in the range 0.508 mm to 0.559 mm (.020" - .022"). In another variation, the sheath guide wire is 0.365 mm (.014") and the inner dilator lumen is in the range 0.406 mm to 0.457 mm (.016" to .018"). The taper is similarly modified, for example the taper length is longer to accommodate a taper from a smaller diameter to the inner diameter of the access sheath, or may comprise two taper angles to provide a smooth transition from the smaller diameter wire to the access sheath without overly lengthening the overall length of the taper.

In some procedures, it is desirable to position the distal tip of the sheath body 222 of the arterial access sheath 220 in the mid to distal cervical, petrous, or cavernous segments of the ICA as described above. These segments have curvature often greater than 90 degrees. In may be desirable to have a sheath dilator with a softer and longer taper, to be able to navigate these bends easily without risk of injury to the arteries. However, in order to insert the sheath through the arterial puncture, the dilator desirably has a certain stiffness and taper to provide the dilating force. In an embodiment, the transcarotid access sheath system 200 is supplied or included in a kit that includes two or more tapered dilators 260A and 260B. The first tapered dilator 260A is used with the arterial access device to gain entry into the artery, and is thus sized and constructed in a manner similar to standard introducer sheath dilators. Example materials that may be used for the tapered dilator include, for example, high density polyethylene, 72D Pebax, 90D Pebax, or equivalent stiffness and lubricity material. A second tapered dilator 260B of the kit may be supplied with the arterial access device with a softer distal section or a distal section that has a lower bending stiffness relative to the distal section of the first tapered dilator. That is, the second dilator has a distal region that is softer, more flexible, or articulates or bends more easily than a corresponding distal region of the first dilator. The distal region of the second dilator thus bends more easily than the corresponding distal region of the first dilator. In an embodiment, the distal section of the first dilator 260A has a bending stiffness in the range of 50 to 100 N-mm² and the distal section of the second dilator 260B has a bending stiffness in the range of 5 to 15 N-mm².

The second dilator 260B (which has a distal section with a lower bending stiffness) may be exchanged with the initial, first dilator such that the arterial access device may be inserted into the internal carotid artery and around curvature in the artery without undue force or trauma on the vessel due to the softer distal section of the second dilator. The distal section of the soft, second dilator may be, for example, 35 or 40D Pebax, with a proximal portion made of, for example 72D Pebax. An intermediate mid portion or portions may be included on the second dilator to provide a smooth transition between the soft distal section and the stiffer proximal section. In an embodiment, one or both dilators may have radiopaque tip markers so that the dilator tip position is visible on fluoroscopy. In one variation, the radiopaque marker is a section of tungsten loaded Pebax or polyurethane which is heat welded to the distal tip of the dilator. Other radiopaque materials may similarly be used to create a radiopaque marker at the distal tip.

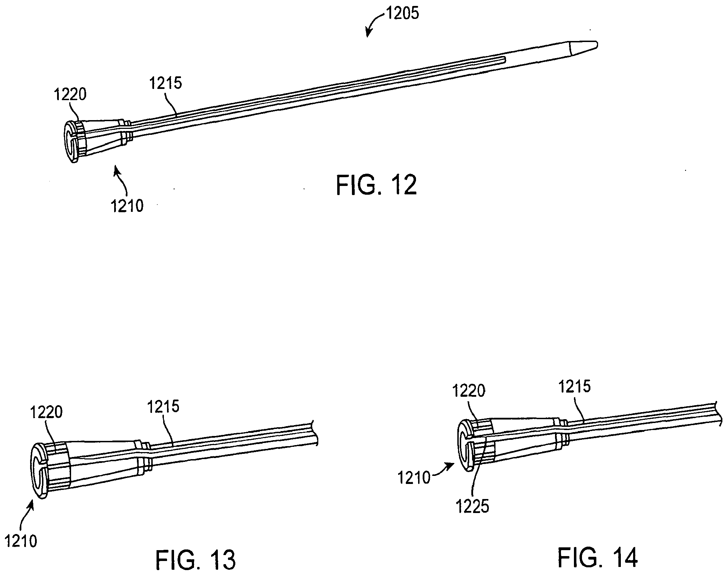

To facilitate exchange of the first dilator for the second dilator, one or both dilators may be configured such that the distal section of the dilator is constructed from a tapered single-lumen tube, but the proximal portion of the dilator and any adaptor on the proximal end has a side opening. FIG. 12 shows an example of a dilator 1205 formed of an elongated member sized and shaped to be inserted into an artery, and a proximal hub 1210. The dilator has a side opening 1215, such as a slot, that extends along at least a portion of the length of the dilator 1205 such as along the elongated body and the proximal hub 1210. In an embodiment, the side opening 1215 is located only on a proximal region of the dilator 1205 and through the proximal hub 1210 although this may vary. The side opening 1215 provides access to an internal lumen of the dilator 1205, such as to insert and/or remove a guidewire into or from the lumen. An annular, movable sleeve 1220 with a slot on one side is located at or near the proximal hub 1210 of the dilator 1205. The sleeve 1220 may be moved, such as via rotation, about a longitudinal axis of the hub 1210, as described below. Note that the distal end of the dilator 1205 has a tapered configuration for dilating tissue.

FIG. 13 shows an enlarged view of the proximal region of the dilator 1205. As mentioned, the dilator 1205 has a side opening 1215 in the form of a slot that extends along the length of the dilator 1205 and the proximal hub 1210. The sleeve 1220 is positioned around the outer periphery of the dilator and is shaped such that it covers at least a portion of the side opening 1215. Thus, the sleeve 1220 can prevent a guidewire positioned inside the dilator 1205 from exiting the dilator via the side opening 1215. As mentioned, the sleeve 1220 is rotatable relative to the dilator 1205 and proximal hub 1210. In the illustrated embodiment, the sleeve 1220 is rotatable about a longitudinal axis of the dilator 1205 although other types of relative movement are within the scope of this disclosure. As shown in FIG. 14, the sleeve 1220 has a slot 1225 that can be aligned with the side opening 1215. When aligned as such, the slot 1225 and side opening 1215 collectively provide an opening for a guidewire to be inserted or removed from the internal lumen of the dilator 1205. The sleeve 1220 can be rotated between the position shown in FIG. 13 (where it covers the side opening 1215) and the position shown in FIG. 14 (where the side opening is uncovered due to the slot 1225 being aligned with the side opening 1215.)

A method of use of this embodiment of an access sheath kit is now described. A sheath guide wire, such as an 0.889 mm (0.035") guidewire, is inserted into the common carotid artery, either using a Modified Seldinger technique or a micropuncture technique. The distal end of the guidewire can be positioned into the internal or external carotid artery, or stop in the common carotid artery short of the bifurcation. The arterial access sheath with the first, stiffer dilator, is inserted over the 0.889 mm (.035") wire into the artery. The arterial access sheath is inserted such that at least 2.5 cm of sheath body 222 is in the artery. If additional purchase is desired, the arterial access sheath may be directed further, and into the internal carotid artery. The first dilator is removed while keeping both the arterial access sheath and the 0.889 mm (.035") wire in place. The side opening 1215 in the proximal portion of the dilator allows the dilator to be removed in a "rapid exchange" fashion such that most of the guidewire outside the access device may be grasped directly during dilator removal. The second dilator is then loaded on to the 0.889 mm (.035") wire and inserted into the sheath. Again, a dilator with a side opening 1215 in the proximal portion of the dilator may be used to allow the 0.889 mm (.035") wire to be grasped directly during guide wire insertion in a "rapid exchange" technique. Once the second dilator is fully inserted into the arterial access device, the arterial access sheath with the softer tipped, second dilator is advanced up the internal carotid artery and around bends in the artery without undue force or concern for vessel trauma. This configuration allows a more distal placement of the arterial access sheath without compromising the ability of the device to be inserted into the artery.

Alternately, one or more standard dilators may be used without side openings. If a standard dilator without a side opening is used, after the access device is inserted into the artery over a guide wire with the first dilator, the first dilator may be removed together with the guidewire, leaving only the access device in place. The second dilator with a guide wire preloaded into the central lumen may be inserted together into the arterial access device. Once fully inserted, the access device and second dilator with softer tip may be advanced distally up the internal carotid artery as above. In this alternate method, the initial guide wire may be used with both dilators, or may be exchanged for a softer tipped guide wire when inserted with the second softer tipped dilator.

In some instances, it may be desirable to insert the access sheath system over an 0.889 mm (.035") wire into the carotid artery, but then exchange the wire to a smaller guidewire, in the range 0.356 mm to 0.457 mm (.014" to .018"). Because the access into the carotid artery may require a steep angle of entry, a wire that can offer good support such as an 0.889 mm (.035") wire may be desirable to initially introduce the access sheath into the CCA. However, once the sheath is in the artery but the user would like to advance it further over a smaller guidewire, it may be desirable to exchange the 0.889 mm (.035") wire for a smaller guide wire. Alternately, the user may exchange both the dilator and 0.889 mm (.035") wire for a softer dilator and smaller guide wire in the range 0.356 mm to 0.457 mm (.014" to .018"). Alternately, the user may wish to position an 0.356 mm (.014") guidewire which he or she will subsequently use to introduce an interventional device, while the sheath and dilator are still in place. The dilator may offer access and support for this guide wire, and in instances of severe access sheath angle may aid in directing the wire away from the posterior wall of the artery so that the wire may be safely advanced into the vascular lumen without risk of luminal injury.

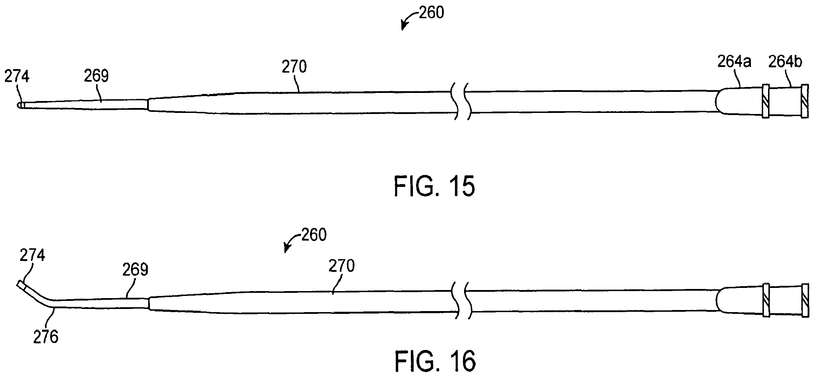

In an embodiment as shown in FIG. 15, the sheath dilator 260 is a two-part dilator assembly, with an inner dilator 269 and an outer dilator 270 that slidably attach to one another in a co-axial arrangement. Both dilators have proximal hubs 264a and 264b. When the two dilators are assembled together, the two hubs 264a and 264b have features which allow them to be locked together, e.g. a snap fit or a threaded fit, so that the two dilators can be handled as one unit. In an embodiment, the inner dilator 269 has a proximal hub 264b which includes a rotating coupler with internal threads that engage external threads on the proximal hub 264a of the outer dilator 270. The inner dilator 269 effectively transforms the dilator assembly from an 0.889 mm or 0.965 mm (.035" or .038") wire compatible dilator to an 0.457 mm or 0.356 mm (.018" or .014") wire compatible dilator, and extends out the distal end of the outer dilator. In an embodiment, shown in FIG. 16, the inner dilator has an angled tip 276 that is bent or angled relative to a longitudinal axis of the remainder of the dilator. In an embodiment, the angle is a 45 degree angle. This angled tip 276 allows the user to direct the guidewire into one or another branch vessel more easily. The inner dilator may have a tapered tip, straight as shown in FIG. 15 or an angled tip as shown in FIG. 16. Alternately, the inner dilator may have a constant outer diameter to the distal end, with a rounded leading edge. In an embodiment, the inner dilator has a radiopaque marker 274 at or near the distal tip to aid in visualization of the dilator under fluoroscopy. In an embodiment, the inner dilator is reinforced to make it more torquable to aid in directing the angled tip in a particular direction. For example the dilator may have a coil or braid reinforcement layer. Once the interventional wire is positioned, the two-part dilator is removed and the wire may then be used to insert interventional devices through the arterial sheath into the artery and advanced to the treatment site.

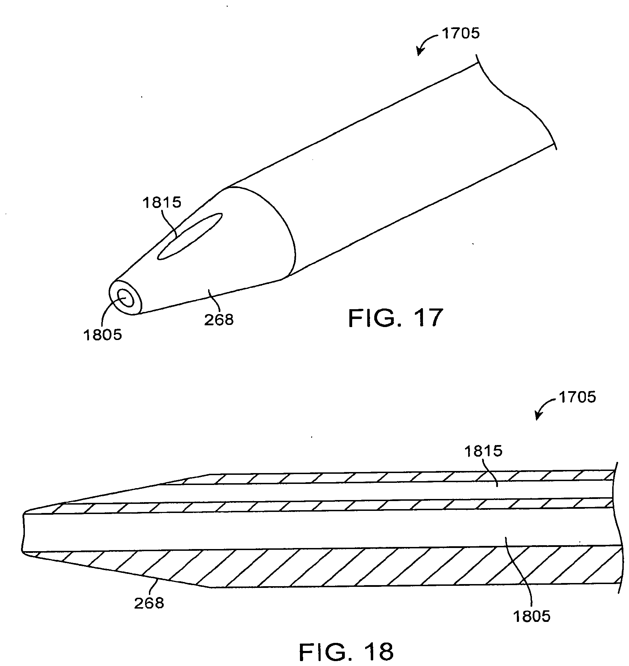

An alternate embodiment, shown in FIG. 17, allows two separate wire sizes to be used with the dilator. This embodiment includes a dilator 1705 with two guide wire internal lumens that extend along the length of the device. FIG. 17 shows the distal end of this embodiment. As seen more clearly in a cross sectional view FIG. 18, one lumen 1805 is configured for an 0.889 mm or 0.965 mm (.035" or .038") guidewire, and the other lumen 1815 is for an 0.356 mm to 0.457 mm (.014" to .018") guide wire. In this embodiment, the larger lumen 1805 is centered around the centerline of the taper 268, whereas the smaller lumen 1815 is offset from the centerline of the taper. In this configuration, the access sheath is introduced into the artery over the larger guidewire, which is positioned in the larger lumen 1805. Once positioned, an interventional wire can be placed through the second lumen 1815. The larger guidewire and dilator are then removed from the access sheath and the interventional wire may then be used to insert interventional devices through the arterial sheath into the artery and advanced to the treatment site as above.

### Sheath Guidewire

Arterial access sheaths are typically introduced into the artery over a sheath guidewire of 0.889 mm or 0.965 mm (.035" or .038") diameter. The inner diameter and taper length of the distal tip of the dilator are sized to fit with such a guidewire. Some sheaths, for example for radial artery access, are sized to accommodate a sheath guidewire of 0.457mm (0.018") diameter, with a corresponding dilator having a distal tip inner diameter and taper length. The sheath guidewire may have an atraumatic straight, angled, or J-tip. The guidewire smoothly transitions to a stiffer segment on the proximal end. This configuration allows atraumatic entry and advancement of the wire into the artery while allowing support for the sheath when the sheath is introduced into the artery over the wire. Typically the transition from the atraumatic tip is about 4 to 9 cm to the stiffer section. The sheath is usually inserted 15 to 20 cm into the artery, so that the stiffer segment of the wire is at the arterial entry site when the sheath is being inserted.

However, in the case of a transcarotid access entry point, the amount of wire that can be inserted is much less than 15 cm before potentially causing harm to the distal vessels. In a case of a transcarotid access for a carotid stent or PTA procedure, it is very important that the wire insertion length is limited, to avoid risk of distal emboli being generated by the sheath guide wire at the site of carotid artery disease. Thus it is desirable to provide a guide wire that is able to provide support for a potentially steep sheath entry angle while being limited in length of insertion. In an embodiment, a transcarotid sheath guidewire has an atraumatic tip section but has a very distal and short transition to a stiffer section. For example, the soft tip section is 1.5 to 2.5 cm, followed by a transition section with length from 3 to 5 cm, followed by a stiffer proximal segment, with the stiffer proximal section comprising the remainder of the wire. In some implementations, the soft tip section (i.e. the distalmost flexible section including the distal tip of the guidewire) is between 1 cm and 2 cm and the transition section positioned between the flexible distalmost section and the more rigid core section extending proximally from the transition section is between 3 cm and 5 cm.

The sheath guidewire may have guide wire markings 318 to help the user determine where the tip of the wire is with respect to the dilator. For example, there may be a marking on the proximal end of the wire corresponding to when the tip of the wire is about to exit the micro access cannula tip. This marking would provide rapid wire position feedback to help the user limit the amount of wire insertion. In another embodiment, the wire may include an additional mark to let the user know the wire has exited the cannula by a set distance, for example 5 cm.

### Micro Access Components

With reference to FIG. 1, a micro access kit 100 for initial transcarotid access includes an access needle 120, an access guidewire 140, and a micro access cannula 160. The micro access cannula 160 includes a body 162 and an inner dilator 168 slidably positioned within a lumen of the body 162. Typically for arterial access, the initial needle puncture may be with a 21G or 22G access needle, or an 18G needle if the Modified Seldinger technique is used. For transcarotid access, it may be desirable to access with an even smaller needle puncture. Percutaneous access of the carotid artery is typically more challenging than of the femoral artery. The carotid artery is a thicker-walled artery, it is surrounded by a tissue sleeve known as the carotid sheath, and it is not anchored down as much by surrounding musculature, therefore the initial needle stick is more difficult and must be done with more force, onto an artery that is less stable, thus increasing the risk of misplaced puncture, arterial dissection, or back wall puncture. A smaller initial needle puncture, for example a 23G or 24G needle, increases the ease of needle entry and reduce these risks. The sheath guidewire should be accordingly sized to fit into the smaller needle, for example a 0.406 mm or 0.356 mm (0.016" or 0.014") wire. The access needle 120 may include a textured surface on the distal end to render it visible on ultrasound, to aid in ultrasound-guided insertion of the needle into the artery. The needle length may be in a range from 4 cm to 8 cm in length.

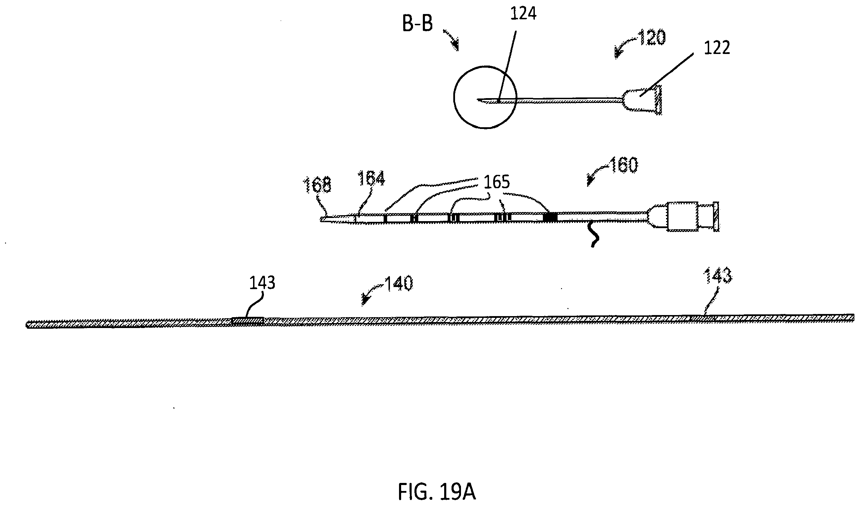

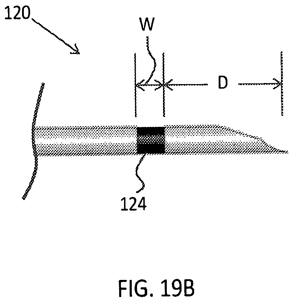

As best shown in FIGs. 19A and 19B, the access needle 120 may also include a visible depth indicator 124 located near the distal end of the needle shaft. The visible depth indicator 124 can be visible to the user without the help of ultrasound or radiography, or other imagining techniques, providing the user with a reference during needle insertion and manipulation of the guidewire during direct insertions into the vessel. The access needle 120, which can be a 21G, 22G, 23G, or 24G needle, can have an elongate shaft defining an inner lumen and coupled to a proximal hub 122. The shaft length from the distal end of needle hub 122 to the distal tip of its shaft can be between 30 mm to about 100 mm. In some implementations, the shaft length is approximately 40 mm or 70 mm. The visible depth indicator 124 can be positioned on the elongate shaft of the needle 120 a distance D away from the distal tip of the needle 120, for example about 3 mm to about 7 mm, or preferably 4.5 mm to 5 mm. In some implementations, the depth indicator 124 can have a width W of approximately 1 mm to about 2 mm such that the indicator 124 is readily visible to the naked eye during use, such as direct access into the common carotid artery (CCA). Presence of the depth indicator 124 reduces the risk that the operator will advance the access needle 120 into or through the opposite wall of the vessel. Advancement of the needle 120 into the vessel can be metered visually by assessing advancement of the depth indicator 124 into the vessel, which is a known distance away from the distal tip. The depth indicator 124 can be created using chemical etch, laser-etching, pad printing or other marking methods.

Access guidewires typically used in micropuncture kits can have relatively long distal floppy sections, for example between 30-50 mm long. However in some applications, such as transcarotid artery revascularization (TCAR), the operator may want to advance the guidewire into the vessel only 30-40 mm to avoid advancing the tip of the guidewire into a diseased section of the artery. For guidewires with long distal floppy sections, inserting only the first 30-40 mm of the guidewire means the supportive section of the guidewire remains outside the vessel and only the non-supportive portion of the guidewire is available for advancing the cannula and dilator. Advancing the microaccess cannula and dilator over the non-supportive section of the guidewire carries a higher risk of causing damage to the inner surface of the vessel. Therefore, the access guidewires described herein have a shorter, distal floppy section that allows for a shorter length to be inserted within the vessel while still ensuring the more rigid proximal support section is available within the vessel for advancement of the cannula and dilator over it.

The guidewire 140 described herein can have a shorter distalmost flexible section terminating in a distal tip, for example, a flexible section that is just 1 cm to 2 cm long. A transition section proximal to the distalmost flexible section transitions proximally in stiffness towards the stiffer core section extending proximally from the transition section. Such micro access guidewires are typically 0.457 mm (0.018") in diameter, with a distalmost flexible section of about 1-2 cm, a transition section of 5-6 cm leading to the stiffer core section extending the remainder of the length of the guidewire. In an embodiment, a transcarotid access guidewire is from 0.356 mm to 0.457 mm (0.014" to 0.018") in diameter, and has a distalmost flexible section of 1 cm, a transition section of 2-3 cm to bring the stiff supportive core section much closer to the distal tip. This will allow the user to have good support for micro access cannula insertion even in steep access angles and limitations on wire insertion length.

As with the sheath guidewire, the micro access guidewire 140 may have at least one guidewire marking or depth indicator 143 visible to the naked eye that is positioned a known distance from the distal tip of the guidewire 140 to help the user determine where the tip of the guidewire 140 is with respect to the vessel as well as other components of the microaccess system 100, e.g. the access needle 120, the micro cannula 160, and/or the inner dilator 168 (see FIGs. 1 and 19A). The depth indicator 143 can provide rapid, wire position feedback without any special visualization techniques such as ultrasound or radiography to help the user limit the amount of wire insertion. For example, a depth indicator 143 can be located at a first location of the guidewire 140 a distance away from the distal tip of the guidewire 140 that when aligned with another portion of the system 100, such as the proximal end of the cannula 160, corresponds to when the tip of the wire 140 is about to exit the micro cannula 160. In another embodiment, the guidewire 140 may include an additional marking 143 to let the user know the guidewire 140 is about to or has exited the dilator by a set distance, for example 5 cm. In another embodiment, the guidewire 140 may include a further marking 143 positioned more distally to let the user know when the guidewire 140 is inserted through the access needle 120 the guidewire 140 is about to exit the access needle 120 or has exited by a set distance. Thus, the guidewire 140 can include a plurality of markings 143 that together provide visual guidance related to the depth of insertion and the relative extension of the guidewire 140 in relation to other system components.

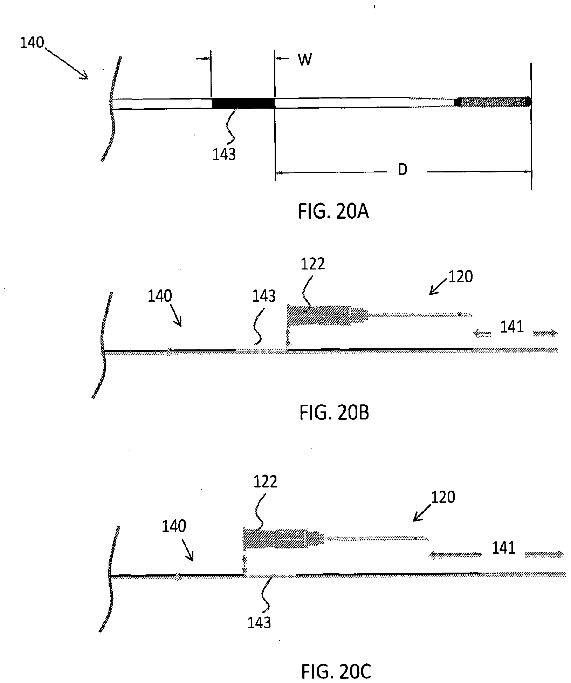

FIGs. 20A-20C illustrate an implementation of using at least one visible depth indicator on the access guidewire 140 that when a portion of the depth indicator is positioned relative to another portion of the system 100 can provide the user with rapid, wire position feedback and metering of the advancement of the guidewire 140 relative to the vessel and other components of the system 100 without needing any special visualization aside from the user's own eyes. This visual reference provides information regarding the distance the guidewire 140 extends beyond the access needle 120, for example, and into the vessel. The guidewire 140 can include at least one visible depth indicator 143 a known distance D from a distal-most end or distal tip of the guidewire 140. The visible depth indicator 143 can have a known width W extending between a proximal edge and a distal edge of the indicator 143. For example, one depth indicator 143 can be 2 cm wide and the distal edge of the depth indicator 143 positioned 10 cm from the distal tip of the guidewire 140 such that the proximal edge of the depth indicator 143 is positioned 12 cm from the distal tip of the guidewire 140. The distance away from the distal tip of the guidewire 140 is measured from the distal tip of the guidewire 140 to a distal edge of the at least one visible depth indicator 143. When the access guidewire is inserted through the inner lumen of the elongate shaft of the access needle 120 aligning the distal edge of the depth indicator 143 with a back end of the proximal hub 122 of the access needle 120 extends the distal tip of the guidewire 140 beyond the distal tip of the elongate shaft a certain extension length. Further advancing the access guidewire 140 through the inner lumen of the elongate shaft of the access needle 120 until the proximal edge is aligned with the back end of the proximal hub 122 of the needle 120 extends the distal tip of the guidewire 140 beyond the distal tip of the elongate shaft of the access needle 120 the extension length plus the width of the depth indicator 143. For example, the needle 120 through which the guidewire 140 is advanced can measure 7 cm from the back end of the proximal hub 122 to the distal tip of the needle. Advancing the guidewire 140 through the access needle 120 until the distal edge of the depth indicator aligns with the back end of the needle hub 122 provides an extension length 141 of the guidewire 140, a portion of the guidewire 140 extending distal to the distal tip of the needle 120, that is 3 cm long (see FIG. 20B). Advancing the guidewire 140 through the needle 120 until the proximal edge of the depth indicator is aligned with the back end of the needle hub 122 provides an extension length 141 of the guidewire 140 distal to the distal tip of the needle 120 that is an additional 2 cm or a total of 5 cm long (see FIG. 20C). It should be appreciated that the guidewire 140 may have multiple discrete markings 143 to indicate various extension lengths 141 and thus depths in the vessel (e.g. 1 cm, 2 cm, 3 cm, etc.) The one or more markings 143 can be created by chemical etch, laser marking, pad printing, or other methods.

Typically, the micro access cannula 160 includes a cannula body 162 configured to receive an inner dilator 168 through its lumen, the dilator 168 having a tapered tip. The inner dilator 168 provides a smooth transition between the cannula 160 and the access guide wire 140. The cannula 160 is sized to receive the 0.889 mm (0.035") wire, with inner diameter in the range about 0.940 mm to 1.041 mm (0.037" to about 0.041"). In an embodiment, a micro access cannula 160 is configured for transcarotid access. For example, the dilator 168 of the cannula 160 may be sized for a smaller 0.356 mm (0.014") access guide wire 140. Additionally, the cannula 160 may have one or more visible depth indicators 165 to aid the user in assessing the amount of insertion of the cannula 160 without any special imaging aside from the naked eye, as will be described in more detail below. A radiopaque material (e.g. barium, bismuth, tungsten) can be added to the entire shaft polymer of the micro access cannula 160 and/or dilator 168 to provide visibility during fluoroscopy. Alternatively or additionally the micro access cannula 160 and/or the dilator 168 can have one or more radiopaque markers 164, for example, at the distal tip of the cannula 162 or dilator 168, to help the user visualize the tip location under fluoroscopy. This is useful for example in cases where the user may want to position the cannula in the ICA or ECA, for example.

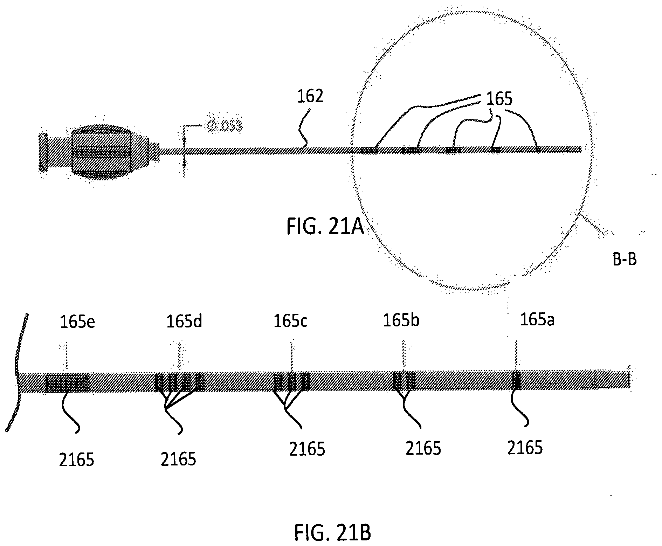

As shown in FIGs. 21A-21B, the microaccess cannula 160 can include a plurality of depth indicators 165 having a known width W and positioned a known distance D from the distal-most tip of the cannula 160. Each of the plurality of visible depth indicators 165 can identify a distance from the distal tip of the cannula 160. The indicators 165 can provide rapid metering of the advancement of the cannula 160 relative to the insertion point into the vessel as well as other components of the system 100 without any special visualization such as ultrasound or radiography. Additionally or alternatively, each of the plurality of visible depth indicators 165 can be formed by a number of marks 2165 such as circumferential rings. Although the marks 2165 are described herein as being circumferential rings it should be appreciated that the marks 2165 need not completely encircle the cannula and can take on other shapes such as dots, dashes, or other visible marks.

Each depth indicator 165 has at least one mark 2165. The number of marks 2165 forming each of the plurality of depth indicators 165 can identify the number of increments that the depth indicator 165 is positioned from the distal tip of the cannula 160. This allows for the total distance to be readily and easily surmised simply by looking at the number of marks 2165 making up each depth indicator 165. In other words, each depth indicator 165 can indicate the distance it is from the distal tip of the cannula 160 based on the number of marks 2165 (whether dots, dashes, bands or circumferential rings) forming the particular depth indicator 165. For example, as best shown in FIGs. 21A-21B, a first depth indicator 165a can be formed by a single circumferential ring 2165 positioned such that a center of the ring 2165 is a first distance from the distal tip (e.g. 10 mm). A second depth indicator 165b can be formed by two circumferential rings 2165 positioned such that a center between the pair of rings 2165 is a second distance from the distal tip (e.g. 20 mm). A third depth indicator 165c can be formed by three circumferential rings 2165 positioned such that a center of the three rings 2165 is a third distance from the distal tip (e.g. 30 mm). A fourth depth indicator 165d can be formed by four of the circumferential rings 2165 positioned such that a center of the four is a fourth distance from the distal tip (e.g. 40 mm). The depth indicators 165 can continue in this pattern thereby metering the distance from the distal tip by standard increments in a way that is readily visible and instantaneously understandable to the user.

It should be appreciated that the measured distance need not be from the distal tip to the center of the rings as described above, but instead can be from the distal tip to a distal edge of the mark(s) 2165 or from the distal tip to a proximal edge of the mark(s) 2165. Additionally, one of the depth indicators 165e can be a solid marker of a known width, the middle of which (or distal edge or proximal edge) identifies a known distance from the distal tip of the cannula 160 that is generally considered an upper depth limit. In some implementations, the micro access cannula 160 is only inserted between 20-30 mm and an upper depth limit can be generally around 50 mm. These distances can vary and more or fewer depth indicators 165 are considered herein. For example, if additional external depth indicators 165 are desired the marker increments can be identified by patterns of mark such as 1 mark, 2 marks, 3 marks, 4 marks, solid band, as described above and then starting over with 1 mark, 2 marks, 3 marks, 4 marks, solid band, and so on. The solid band breaking up the mark pattern provides a quick and easily identifiable indication that the fifth marker increment in the series has been reached without requiring a user to count too many rings. For example, more than 4 marks per depth indicator 165 can be tedious to differentiate and lead to reading errors by the user. Additionally, an increment size of 10 mm is generally easy to calculate quickly. It should be appreciated, however, that other increment sizes are considered such as 5 mm, 15 mm, or even 20 mm for longer cannulas. One or more of the depth indicators 165 can be distinguishable based on its color as well as by the number and/or pattern of marks. For example, a first mark can be a first color, a second mark can be a second color, a third mark can be third color and so on. The marks can be easily distinguishable colors indicating depth of insertion (e.g. white, yellow, orange, red, green, blue, black, etc.) The marks can be within a similar color family, but have an increasing intensity or tone to distinguish the depth of insertion (e.g. a first mark being pale pink towards a last mark being deep red).

The depth indicators 165 may be created by pad printing, laser marking, additions of colorant to the cannula material, or other methods. Other components of the micro access kit 100 can include similar metering markings as described above. For example, the depth indicators 143 of the guidewire 140 may also be metered to indicate distance from the distal tip depending on the number of marks (or color, size, shape) per indicator 143. As described above, the sheath body 220 and/or the sheath stopper 1105 can incorporate one or more markings that provide an indication of the depth of insertion of the sheath 220.

### Exemplary Kits:

Any or all of the devices described above may be provided in kit form to the user such that one or more of the components of the systems are included in a common package or collection of packages. An embodiment of an access sheath kit comprises an access sheath, sheath dilator, and sheath guidewire all configured for transcarotid access as described above.

In an embodiment, a micro access kit comprises an access needle, a micro access guide wire, and a micro access cannula and dilator wherein the guidewire is 0.356 mm (0.014") and the micro access cannula and dilator are sized to be compatible with the 0.356 mm (0.014") guide wire.

In an embodiment, an access kit comprises the access sheath, sheath dilator, sheath guide wire, access needle, micro access guide wire and micro access cannula and dilator, all configured for transcarotid access.

In an alternate embodiment, the access guidewire is also used as the sheath guide wire. In this embodiment, the access kit comprises an access needle, access guide wire, access sheath and dilator. The sheath and dilator use the access guide wire to be inserted into the vessel, thereby avoiding the steps required to exchange up to a larger sheath guidewire. In this embodiment, the dilator taper length and inner lumen is sized to be compatible with the smaller access guide wire. In one embodiment, the access guide wire is 0.457 mm (0.018"). In an alternate embodiment the access guide wire is 0.406 mm (0.016"). In an alternate embodiment, the access guide wire is 0.356 mm (0.014").

### Exemplary Methods:

There are now described exemplary methods of use for a transcarotid access system. In an exemplary transcarotid procedure to treat a carotid artery stenosis, the user starts by performing a cut down to the common carotid artery. The user then inserts an access needle 120 into the common carotid artery at the desired access site. An access guide wire 140 with a taper configured for transcarotid access is inserted through the needle into the common carotid artery and advanced into the CCA. The access needle 120 is removed and a micro access cannula 160 is inserted over the wire 140 into the CCA. The micro access cannula is inserted a desired depth using the marks 165 on the cannula as a guide, to prevent over insertion.

The user removes the cannula inner dilator 168 and guide wire 140, leaving the cannula 162 in place. If desired, the user performs an angiogram through the cannula 162. The user then places sheath guide wire 300 through the cannula, using guide wire markings 318 to aid in inserting the wire to a desired insertion length. The cannula 162 is removed from the guidewire and the access sheath 220 and sheath dilator 260 are inserted as an assembly over the sheath guidewire 300 into the CCA. The sheath stopper flange 1115 of the sheath stopper 1105 limits the insertion length of the arterial sheath. Once positioned, the dilator 260 and guidewire 300 are removed. The sheath is then sutured to the patient using the securing eyelets 234, ribs 236, and/or suture grooves 1120a. An interventional procedure is then performed by introduction of interventional devices through hemostasis valve 226 on the proximal end of the arterial sheath and to the desire treatment site. Contrast injections may be made as desired during the procedure via the flush arm 228 on the arterial sheath 220.

Alternately, the user inserts an access needle 120 directly into the common carotid artery at the desired access site visually monitoring a depth indicator 124 on the needle 120. Vessel wall thickness is typically 1-2 mm and the vessel diameter of the common carotid artery is typically 8-9 mm. Thus, maintaining the depth indicator 124 positioned 5 mm from the distal tip in view outside the vessel ensures the distance the needle is inserted will penetrate the wall thickness fully without contacting the back side of the vessel wall.

Alternately or additionally, an access guidewire 140 configured for transcarotid access is inserted through the hub 122 of the needle 120 until a portion of the depth marker 143 aligns with another portion of the needle 120 identifying a desired amount of extension length 141 of the guidewire 140 is positioned within the common carotid artery. For example, the proximal edge of the depth marker 143 on the guidewire 140 can be aligned with the back end of the needle hub 122 indicating 5 cm of wire has been advanced beyond the distal tip of the needle 120 into the common carotid artery. Alternatively, the distal edge of the depth marker 143 is aligned with the back end of the needle hub 122 indicating 3 cm of wire beyond the distal tip of the needle into the common carotid artery.

Leaving the access guidewire 140 in place, the access needle 120 is carefully withdrawn from the vessel. The micropuncture cannula 160 having the inner dilator 168 extending through its lumen is advanced over the access guidewire 140 into the vessel through the puncture. The depth markings 165 of the micropuncture cannula 160 are monitored visually by the user to confirm desired depth of micropuncture cannula insertion and to prevent over-insertion. The micropuncture cannula 160 is left in place once desired depth is reached, and the dilator 168 and access guidewire 140 are carefully withdrawn.

Alternately, the sheath guidewire 300 is placed into the CCA via a single needle puncture with a larger access needle, for example an 18G needle. In this embodiment, the access cannula and access guide wire are not needed. This embodiment reduces the number of steps required to access the artery, and in some circumstances may be desirable to the user.

Alternately, the sheath dilator is a two-part sheath dilator assembly 260 as shown in FIG. 15, with an inner dilator 269 and an outer dilator 270. The outer dilator 270 is configured to receive an 0.889 mm (.035") sheath guide wire 300 and to provide a smooth transition from the 0.889 mm (.035") wire to the access sheath 220. The inner dilator 269 is configured to receive a smaller guide wire in the range 0.356 to 0.457 mm (.014" to .018") and to provide a smooth transition from the smaller guide wire to the outer dilator 270. Once the sheath guidewire is positioned in the CCA, the access sheath and outer sheath dilator 270 are inserted over an 0.889 mm (.035") sheath guidewire 300 into the CCA. The guidewire is then removed and an inner sheath dilator 269 is inserted into the outer sheath dilator. In an embodiment, the inner sheath dilator has an angled tip 276 as seen in FIG. 16. An interventional 0.356 mm (.014") guide wire is inserted through the inner sheath dilator and is directed to the target treatment site using the angled tip to aid in guide wire positioning. Alternately, the inner sheath dilator has a straight tip and is used to aid in positioning the guide wire safely into the CCA. Once the 0.356 mm (.014") wire is positioned at or across the target treatment site, the sheath dilator 260 and sheath 0.889 mm (.035") guide wire 300 are then removed, and the intervention proceeds.

In an alternate embodiment, the sheath dilator is a two lumen sheath dilator 1705. In this embodiment, the sheath and dilator are inserted over the sheath guide wire 300, with the sheath guidewire positioned in the larger lumen 1805 of dilator 1705. Once the sheath and dilator is in place, an interventional 0.356 mm (.014") guide wire is positioned through the smaller lumen 1815. The dilator provides distal support and maintains the position of the sheath tip in the axial direction of the vessel lumen, thus allowing a potentially safer and easier advancement of the 0.356 mm (.014") wire than if the dilator were removed and the sheath tip was directed at least partially towards to posterior wall of the artery. Once the 0.356 mm (.014") wire is positioned at or across the target treatment site, the sheath dilator 1705 and sheath guide wire 0.889 mm (.035") are then removed, and the intervention proceeds.

In yet another embodiment, it may be desirable to occlude the CCA during the intervention to minimize antegrade flow of emboli. In this embodiment, the occlusion step may be performed via vascular surgical means such as with a vessel loop, tourniquet, or vascular clamp. In an alternate embodiment, the access sheath 220 has an occlusion element such as an occlusion balloon 805 on the distal tip. In this embodiment, the balloon is inflated when CCA occlusion is desired. In a further variant, while the CCA is occluded either surgically or via balloon occlusion, it may be desirable to connect the arterial sheath to a flow shunt, for example to create a reverse flow system around the area of the treatment site to minimize distal emboli. In this embodiment, the arterial sheath 220 has a Y connection to a flow line 1005. The flow line 1005 may be connected to a return site with a pressure lower than arterial pressure to create a pressure gradient that results in reverse flow through the shunt, for example an external reservoir or a central venous return site like the femoral vein or the internal jugular vein. Alternately, the flow line may be connected to an aspiration source such as an aspiration pump or syringe.

In another embodiment, a transcarotid access system is used to perform a percutaneous neurointerventional procedure. In this embodiment, the user performs a percutaneous puncture of the common carotid artery CCA with an access needle 120 at the desired access site. Ultrasound may be used to accurately identify a suitable access site and guide the needle puncture. An access guide wire 140 is inserted through the needle into the common carotid artery and advanced into the CCA. The access needle 120 is removed and a micro access cannula 160 is inserted over the wire 140 into the CCA. The user removes the cannula inner dilator 168 and guide wire 140, leaving the cannula 162 in place. If desired, the user performs an angiogram through the cannula 162. The user then places sheath guide wire 300 through the cannula, using guide wire markings 318 to aid in desired insertion length. The cannula 162 is removed from the guidewire and the access sheath 220 and sheath dilator 260 are inserted as an assembly over the sheath guidewire 300 into the CCA.

Alternately, the smaller access guide wire 140 is used to position the access sheath 220 and sheath dilator 260 into the CCA. In this embodiment, the sheath dilator tapered tip 268 has been configured to transition smoothly from the access guide wire 140 to the access sheath 220. In one variant, the access needle is 21G and the access guide wire is 0.457 mm (0.018"). In another variant, the access needle is 24G and the access guide wire is 0.356 mm (0.014"). Once the sheath is placed, the guide wire and sheath dilator are removed and an interventional procedure is then performed by introduction of interventional devices through hemostasis valve 226 on the proximal end of the arterial sheath and to the desire treatment site. Contrast injections may be made as desired during the procedure via the flush arm 228 on the arterial sheath 220.

Alternately, it may be desirable once the sheath is placed in the CCA to advance it further into the ICA, for example in the mid to distal cervical ICA, petrous ICA or further distally. In this embodiment, the sheath dilator may be replaced with a softer sheath dilator so that the sheath may be advanced without risk of damaging the distal ICA. In this embodiment, the softer dilator has a distal radiopaque marker so that the user may easily visualize the leading edge of the sheath and dilator assembly during positioning of the sheath. Once the access sheath is positioned, the dilator and sheath guide wire may be removed and the intervention can proceed. Alternately, once the sheath is placed in the CCA, the 0.889 mm (.035") guide wire may be removed and an inner dilator with a smaller guidewire in the range 0.356 mm to 0.457 mm (.014" to .018") may be inserted into sheath dilator. The sheath dilator assembly with the inner dilator and smaller guide wire may be then positioned more distally in the ICA with reduced risk of vessel trauma.

In an embodiment, it may be desirable to occlude the CCA or ICA during portions of the procedure to reduce the chance of distal emboli flowing to the brain. In this embodiment, the CCA or ICA is occluded by means of an occlusion balloon 805 on the access sheath 220. It may also be desirable to connect the arterial sheath to a flow shunt, for example to create a reverse flow system around the area of the treatment site to minimize distal emboli. In this embodiment, the arterial sheath 220 has a Y connection to a flow line 1005. The flow line may be connected to a return site with a pressure lower than arterial pressure to create a pressure gradient that results in reverse flow through the shunt. Alternately, the flow line may be connected to an aspiration source such as an aspiration pump or syringe.

The scope of protection for the invention is defined by the appended claims.

## Claims

1. A micropuncture kit for direct access into a lumen of a surgically exposed vessel using direct visual guidance, the kit comprising:
a micropuncture access needle (120) comprising a proximal hub coupled to an elongate shaft defining an inner lumen and a visible depth indicator (124) positioned on the elongate shaft a distance away from a distal tip of the elongate shaft, wherein the visible depth indicator (124) of the micropuncture access needle (120) provides an indication as to whether a microcatheter, when inserted into the vessel, will penetrate a wall thickness of the vessel without contacting a back side of a vessel wall of the blood vessel;
an access guidewire (140) sized to be received through the inner lumen of the micropuncture access needle (120), the guidewire (140) comprising a distal tip and at least one visible depth indicator (143) positioned on the access guidewire (140) a distance away from the distal tip of the guidewire (140); and
a microaccess cannula (160) comprising an elongate body defining an inner lumen and a plurality of visible depth indicators (165) formed on the elongate body, wherein each of the plurality of visible depth indicators (165) identifies a distance from a distal tip of the cannula (160)
wherein alignment of a first visible depth indicator of the at least one visible depth indicator (143) positioned on the access guidewire (140) with a proximal edge of the cannula (160) provides a visual indication to a user that the distal tip of the guidewire (140) is aligned with the distal tip of the cannula (162);
wherein alignment of a second visible depth indicator of the at least one visible depth indicator (143) positioned on the access guidewire (140) with a proximal edge of the cannula provides a visual indication to a user that the distal tip of the guidewire (140) is aligned with the distal tip of the micropuncture access needle (120);
wherein alignment of a third visible depth indicator of the at least one visible depth indicator (143) positioned on the access guidewire (140) with a proximal edge of the cannula provides a visual indication to a user that the distal tip of the guidewire (140) is positioned distally of the distal tip of the micropuncture access needle (120) by a predetermined distance indicated by the third visible depth indicator.

2. The kit of claim 1, wherein the access guidewire (140) comprises a distalmost flexible section including the distal tip of the guidewire, a transition section proximal to the distalmost flexible section, and a stiffer core section extending proximally from the transition section.

3. The kit of claim 2, wherein the distalmost flexible section is between 1 cm and 2 cm, and the transition section is between 2 cm and 3 cm.

4. The kit of claim 2 or 3, wherein the transition section and the core section are configured to support the microaccess cannula (160) inserted into the vessel.

5. The kit of any preceding claim, wherein the guidewire (140) is in a range of 0.356 mm to 0.457 mm (0.014" to 0.018") outer diameter and wherein the micropuncture needle (120) is in a range from 0.80 mm to 0.55 mm (21G to 24G).

6. The kit of any preceding claim, wherein the at least one visible depth indicator (143) positioned on the access guidewire (140) has a proximal edge, a distal edge and a width extending between the proximal edge and the distal edge, optionally wherein the distance away from the distal tip of the guidewire (140) is measured from the distal tip to the distal edge of the at least one visible depth indicator (143).

7. The kit of claim 6, wherein inserting the access guidewire (140) through the inner lumen of the elongate shaft until the distal edge is aligned with a back end of the proximal hub of the access needle (122) extends the distal tip of the guidewire beyond the distal tip of the elongate shaft an extension length.

8. The kit of claim 6 or 7, wherein advancing the access guidewire (140) through the inner lumen of the elongate shaft until the proximal edge is aligned with the back end of the proximal hub of the access needle (122) extends the distal tip of the guidewire beyond the distal tip of the elongate shaft the extension length plus the width of the at least one visible depth indicator (143).

9. The kit of any preceding claim, wherein the extension length is 3 cm and the width of the at least one visible depth indicator 2 cm.

10. The kit of any preceding claim, wherein the at least one visible depth indicator (124) of the access needle (120) is a chemical-etched marker, a laser-etched marker, or a pad printed marker.

11. The kit of claim 1, wherein there are a plurality of visible depth indicators (165), each formed by a number of marks, wherein the number of marks identifies a number of increments from the distal tip of the cannula (160).

12. The kit of claim 11, wherein each increment is 10 mm and the number of marks is at least one mark.

13. The kit of claim 11 or 12, wherein a first of the plurality of visible depth indicators (165) on the cannula comprises one mark and the first visible depth indicator is 10 mm away from the distal tip of the cannula (160).

14. The kit of claim 13, wherein a second of the plurality of visible depth indicators (165) on the cannula comprises two marks and the second visible depth indicator is 20 mm away from the distal tip of the cannula (160),
optionally a third of the plurality of visible depth indicators on the cannula comprises three marks and the third visible depth indicator is 30 mm away from the distal tip of the cannula (160),
further optionally a fourth of the plurality of visible depth indicators on the cannula comprises four marks and the fourth visible depth indicator is 40 mm away from the distal tip of the cannula (160),
and yet further optionally a fifth of the plurality of visible depth indicators on the cannula is 50 mm away from the distal tip of the cannula (160) and comprises a solid band having a width that is greater than a width of one mark.

15. The kit of any preceding claim, wherein the distance the visible depth indicator of the access needle (124) is from the distal tip of the elongate shaft is between 3 mm and 7 mm.

## Patentansprüche

1. Mikropunktionskit für den direkten Zugang zu einem Lumen eines operativ freigelegten Gefäßes unter Verwendung direkter visueller Führung, wobei das Kit Folgendes umfasst:
eine Mikropunktionszugangsnadel (120), die eine proximale Nabe, die mit einem länglichen Schaft gekoppelt ist, der ein inneres Lumen definiert, und einen sichtbaren Tiefenindikator (124), der an dem länglichen Schaft in einem Abstand von einer distalen Spitze des länglichen Schafts entfernt positioniert ist, umfasst, wobei der sichtbare Tiefenindikator (124) der Mikropunktionszugangsnadel (120) eine Angabe darüber bereitstellt, ob ein Mikrokatheter, wenn in das Gefäß eingesetzt, eine Wanddicke des Gefäßes durchdringen wird, ohne eine Rückseite einer Gefäßwand des Blutgefäßes zu berühren;
einen Zugangsführungsdraht (140), der so bemessen ist, dass er durch das innere Lumen der Mikropunktionszugangsnadel (120) aufgenommen werden kann, wobei der Führungsdraht (140) eine distale Spitze und mindestens einen sichtbaren Tiefenindikator (143), der auf dem Zugangsführungsdraht (140) in einem Abstand von der distalen Spitze des Führungsdrahts (140) entfernt positioniert ist, umfasst; und
eine Mikrozugangskanüle (160), die einen länglichen Körper, der ein inneres Lumen definiert, und eine Vielzahl von sichtbaren Tiefenindikatoren (165), die an dem länglichen Körper ausgebildet sind, umfasst, wobei jeder der Vielzahl von sichtbaren Tiefenindikatoren (165) einen Abstand zu einer distalen Spitze der Kanüle (160) identifiziert
wobei eine Ausrichtung eines ersten sichtbaren Tiefenindikators von dem mindestens einen sichtbaren Tiefenindikator (143), der auf dem Zugangsführungsdraht (140) positioniert ist, mit einer proximalen Kante der Kanüle (160) eine visuelle Angabe für einen Benutzer darüber bereitstellt, dass die distale Spitze des Führungsdrahts (140) mit der distalen Spitze der Kanüle (162) ausgerichtet ist;
wobei eine Ausrichtung eines zweiten sichtbaren Tiefenindikators von dem mindestens einen sichtbaren Tiefenindikator (143), der auf dem Zugangsführungsdraht (140) positioniert ist, mit einer proximalen Kante der Kanüle eine visuelle Angabe für einen Benutzer darüber bereitstellt, dass die distale Spitze des Führungsdrahts (140) mit der distalen Spitze der Mikropunktionszugangsnadel (120) ausgerichtet ist;
wobei eine Ausrichtung eines zweiten sichtbaren Tiefenindikators von dem mindestens einen sichtbaren Tiefenindikator (143), der auf dem Zugangsführungsdraht (140) positioniert ist, mit einer proximalen Kante der Kanüle eine visuelle Angabe für einen Benutzer darüber bereitstellt, dass die distale Spitze des Führungsdrahts (140) um einen vorbestimmten Abstand, der durch den dritten visuellen Tiefenindikator angegeben wird, distal von der distalen Spitze der Mikropunktionszugangsnadel (120) positioniert ist.

2. Kit nach Anspruch 1, wobei der Zugangsführungsdraht (140) einen distalsten flexiblen Abschnitt, der die distale Spitze des Führungsdrahts beinhaltet, einen Übergangsabschnitt, der proximal zu dem distalsten flexiblen Abschnitt ist, und einen steiferen Kernabschnitt, der sich proximal von dem Übergangsabschnitt erstreckt, umfasst.

3. Kit nach Anspruch 2, wobei der distalste flexible Abschnitt zwischen 1 cm und 2 cm beträgt, und der Übergangsabschnitt zwischen 2 cm und 3 cm beträgt.

4. Kit nach Anspruch 2 oder 3, wobei der Übergangsabschnitt und der Kernabschnitt so konfiguriert sind, dass sie die in das Gefäß eingeführte Mikrozugangskanüle (160) stützen.

5. Kit nach einem der vorhergehenden Ansprüche, wobei der Führungsdraht (140) in einem Außendurchmesserbereich von 0,356 mm bis 0,457 mm (0,014" bis 0,018") liegt, und wobei die Mikropunktionsnadel (120) in einem Bereich von 0,80 mm bis 0,55 mm (21G bis 24G) liegt.

6. Kit nach einem der vorhergehenden Ansprüche, wobei der mindestens eine sichtbare Tiefenindikator (143), der auf dem Zugangsführungsdraht (140) positioniert ist, eine proximale Kante, eine distale Kante und eine Breite, die sich zwischen der proximalen Kante und der distalen Kante erstreckt, aufweist, wobei optional der Abstand weg von der distalen Spitze des Führungsdrahts (140) von der distalen Spitze zu der distalen Kante des mindestens einen sichtbaren Tiefenindikators (143) gemessen wird.

7. Kit nach Anspruch 6, wobei das Einführen des Zugangsführungsdrahts (140) durch das innere Lumen des länglichen Schafts, bis die distale Kante mit einem hinteren Ende der proximalen Nabe der Zugangsnadel (122) ausgerichtet ist, die distale Spitze des Führungsdrahts um eine Erstreckungslänge über die distale Spitze des länglichen Schafts hinaus verlängert.

8. Kit nach Anspruch 6 oder 7, wobei das Vorschieben des Zugangsführungsdrahts (140) durch das innere Lumen des länglichen Schafts, bis die proximale Kante mit dem hinteren Ende der proximalen Nabe der Zugangsnadel (122) ausgerichtet ist, die distale Spitze des Führungsdrahts über die distale Spitze des länglichen Schafts hinaus um die Erstreckungslänge zuzüglich der Breite des mindestens einen sichtbaren Tiefenindikators (143) verlängert.

9. Kit nach einem der vorhergehenden Ansprüche, wobei die Erstreckungslänge 3 cm beträgt und die Breite des mindestens einen sichtbaren Tiefenindikators 2 cm beträgt.

10. Kit nach einem der vorhergehenden Ansprüche, wobei der mindestens eine sichtbare Tiefenindikator (124) der Zugangsnadel (120) eine chemisch geätzte Markierung, eine lasergeätzte Markierung oder eine Tampondruckmarkierung ist.

11. Kit nach Anspruch 1, wobei es eine Vielzahl von sichtbaren Tiefenindikatoren (165) gibt, die jeweils durch eine Anzahl von Markierungen gebildet sind, wobei die Anzahl von Markierungen eine Anzahl von Inkrementen ausgehend von der distalen Spitze der Kanüle (160) bestimmt.

12. Kit nach Anspruch 11, wobei jedes Inkrement 10 mm beträgt und die Anzahl der Markierungen mindestens eine Markierung ist.

13. Kit nach Anspruch 11 oder 12, wobei ein erster der Vielzahl von sichtbaren Tiefenindikatoren (165) an der Kanüle eine Markierung umfasst und der erste sichtbare Tiefenindikator 10 mm von der distalen Spitze der Kanüle (160) entfernt ist.

14. Kit nach Anspruch 13, wobei ein zweiter der Vielzahl von sichtbaren Tiefenindikatoren (165) an der Kanüle zwei Markierungen umfasst und der zweite sichtbare Tiefenindikator 20 mm von der distalen Spitze der Kanüle (160) entfernt ist,
wobei optional ein dritter der Vielzahl von sichtbaren Tiefenindikatoren an der Kanüle drei Markierungen umfasst und der dritte sichtbare Tiefenindikator 30 mm von der distalen Spitze der Kanüle (160) entfernt ist,
wobei weiter optional ein vierter der Vielzahl von sichtbaren Tiefenindikatoren an der Kanüle viert Markierungen umfasst und der vierte sichtbare Tiefenindikator 40 mm von der distalen Spitze der Kanüle (160) entfernt ist,
und wobei noch weiter optional ein fünfter der Vielzahl von sichtbaren Tiefenindikatoren auf der Kanüle 50 mm von der distalen Spitze der Kanüle (160) entfernt ist und ein durchgehendes Band umfasst, das eine Breite aufweist, die größer als eine Breite einer Markierung ist.

15. Kit nach einem der vorhergehenden Ansprüche, wobei der Abstand, den der sichtbare Tiefenindikator der Zugangsnadel (124) von der distalen Spitze des länglichen Schafts entfernt ist, zwischen 3 mm und 7 mm beträgt.

## Revendications

1. Kit de microperforation pour un accès direct à la lumière d'un vaisseau exposé chirurgicalement, à l'aide d'un guidage visuel direct, le kit comprenant :
une aiguille d'accès par microperforation (120) comprenant une embase proximale couplée à une tige allongée définissant une lumière interne et un indicateur de profondeur visible (124) situé sur la tige allongée à une certaine distance d'une pointe distale de la tige allongée, ledit indicateur de profondeur visible (124) de l'aiguille d'accès par microperforation (120) offrant une indication du fait qu'un microcathéter, une fois inséré dans le vaisseau, pourra pénétrer une épaisseur de paroi du vaisseau sans entrer en contact avec le côté arrière d'une paroi du vaisseau sanguin ;
un fil-guide d'accès (140) dimensionné pour être reçu à travers la lumière interne de l'aiguille d'accès par microperforation (120), le fil-guide (140) comprenant un bout distal et au moins un indicateur de profondeur visible (143) situé sur le fil-guide d'accès (140) à une certaine distance du bout distal du fil-guide (140) ; et
une canule de microaccès (160) comprenant un corps allongé définissant une lumière interne et une pluralité d'indicateurs de profondeur visibles (165) formés sur le corps allongé, chacun des indicateurs de la pluralité d'indicateurs de profondeur visibles (165) identifiant une distance le séparant d'une pointe distale de la canule (160) ;
l'alignement d'un premier indicateur de profondeur visible faisant partie de l'au moins un indicateur de profondeur visible (143) situé sur le fil-guide d'accès (140) avec un bord proximal de la canule (160) offrant une indication visuelle à un utilisateur du fait que le bout distal du fil-guide (140) est aligné avec la pointe distale de la canule (162) ;
l'alignement d'un deuxième indicateur de profondeur visible faisant partie de l'au moins un indicateur de profondeur visible (143) situé sur le fil-guide d'accès (140) avec un bord proximal de la canule offrant une indication visuelle à un utilisateur du fait que le bout distal du fil-guide (140) est aligné avec la pointe distale de l'aiguille d'accès par microperforation (120) ;
l'alignement d'un troisième indicateur de profondeur visible faisant partie de l'au moins un indicateur de profondeur visible (143) situé sur le fil-guide d'accès (140) avec un bord proximal de la canule offrant une indication visuelle à un utilisateur du fait que le bout distal du fil-guide (140) est situé distalement par rapport à la pointe distale de l'aiguille d'accès par microperforation (120) selon une distance prédéterminée indiquée par le troisième indicateur de profondeur visible.

2. Kit selon la revendication 1, dans lequel le fil-guide d'accès (140) comprend une section flexible la plus distale comportant le bout distal du fil-guide, une section de transition proximale à la section flexible la plus distale, et une section d'âme plus rigide s'étendant proximalement à partir de la section de transition.

3. Kit selon la revendication 2, dans lequel la section flexible la plus distale fait entre 1 cm et 2 cm, et la section de transition fait entre 2 cm et 3 cm.

4. Kit selon la revendication 2 ou 3, dans lequel la section de transition et la section d'âme sont conçues pour supporter la canule de microaccès (160) insérée dans le vaisseau.

5. Kit selon l'une quelconque des revendications précédentes, dans lequel le fil-guide (140) a un diamètre externe compris dans une plage de 0,356 mm à 0,457 mm (0,014 pouce à 0,018 pouce) et dans lequel l'aiguille de microperforation (120) est comprise dans une plage de 0,80 mm à 0,55 mm (21G à 24G).

6. Kit selon l'une quelconque des revendications précédentes, dans lequel l'au moins un indicateur de profondeur visible (143) situé sur le fil-guide d'accès (140) a un bord proximal, un bord distal et une largeur s'étendant entre le bord proximal et le bord distal, la distance par rapport au bout distal du fil-guide (140) étant éventuellement mesurée à partir du bout distal et jusqu'au bord distal de l'au moins un indicateur de profondeur visible (143).

7. Kit selon la revendication 6, dans lequel l'insertion du fil-guide d'accès (140) dans la lumière interne de la tige allongée jusqu'à ce que le bord distal soit aligné avec une extrémité arrière de l'embase proximale de l'aiguille d'accès (122) prolonge le bout distal du fil-guide au-delà de la pointe distale de la tige allongée sur une longueur d'extension.

8. Kit selon la revendication 6 ou 7, dans lequel l'avancée du fil-guide d'accès (140) à travers la lumière interne de la tige allongée jusqu'à ce que le bord proximal soit aligné avec l'extrémité arrière de l'embase proximale de l'aiguille d'accès (122) prolonge le bout distal du fil-guide au-delà de la pointe distale de la tige allongée sur la longueur d'extension plus la largeur de l'au moins un indicateur de profondeur visible (143).

9. Kit selon l'une quelconque des revendications précédentes, dans lequel la longueur d'extension est de 3 cm et la largeur de l'au moins un indicateur de profondeur visible est de 2 cm.

10. Kit selon l'une quelconque des revendications précédentes, dans lequel l'au moins un indicateur de profondeur visible (124) de l'aiguille d'accès (120) est un marqueur gravé chimiquement, un marqueur gravé au laser, ou un marqueur imprimé au tampon.

11. Kit selon la revendication 1, dans lequel il existe une pluralité d'indicateurs de profondeur visibles (165), chacun étant formé par un certain nombre de marques, ledit nombre de marques identifiant un certain nombre d'incréments par rapport à la pointe distale de la canule (160).

12. Kit selon la revendication 11, dans lequel chaque incrément est de 10 mm et le nombre de marques est d'au moins une marque.

13. Kit selon la revendication 11 ou 12, dans lequel un premier indicateur de la pluralité d'indicateurs de profondeur visibles (165) sur la canule comprend une marque et le premier indicateur de profondeur visible se trouve à 10 mm de la pointe distale de la canule (160).

14. Kit selon la revendication 13, dans lequel un deuxième indicateur de la pluralité d'indicateurs de profondeur visibles (165) sur la canule comprend deux marques et le deuxième indicateur de profondeur visible se trouve à 20 mm de la pointe distale de la canule (160),
éventuellement un troisième indicateur de la pluralité d'indicateurs de profondeur visibles sur la canule comprend trois marques et le troisième indicateur de profondeur visible se trouve à 30 mm de la pointe distale de la canule (160),
éventuellement en outre un quatrième indicateur de la pluralité d'indicateurs de profondeur visibles sur la canule comprend quatre marques et le quatrième indicateur de profondeur visible se trouve à 40 mm de la pointe distale de la canule (160),
et éventuellement encore un cinquième indicateur de la pluralité d'indicateurs de profondeur visibles sur la canule se trouve à 50 mm de la pointe distale de la canule (160) et comprend une bande continue ayant une largeur supérieure à la largeur d'une seule marque.

15. Kit selon l'une quelconque des revendications précédentes, dans lequel la distance entre l'indicateur de profondeur visible de l'aiguille d'accès (124) et la pointe distale de la tige allongée est comprise entre 3 mm et 7 mm.
